# EUROPEAN PATENT APPLICATION

(11) **EP 2 107 054 A1**
(43) Date of publication of application: **07.10.2009**
(21) Application number: 08006651.7
(22) Date of filing: 01.04.2008
(51) Int. Cl.: C07D 233/88, C07D 277/40, C07D 277/46, C07D 307/68, C07D 413/14, C07D 417/04, C07D 417/10, C07D 417/12, C07D 417/14, A61K 31/422, A61K 31/427, A61K 31/433

(54) **Antiproliferative compounds and therapeutic uses thereof**

(71) Applicant: Università Degli Studi Di Milano - Bicocca, 20126 Milano (IT); UNIVERSITE DE GENEVE, 1211 Geneve 4 (CH); UNIVERSITE CLAUDE BERNARD - LYON 1, 69622 Villeurbanne Cédex (FR)
(72) Inventor: Gambacorti Passerini, Carlo, 20052 (MI) (IT); Gunby, Rosalind Helen, 20144 Milano (IT); Zambon, Alfonso, 4325 Venezia (IT); Scapozza, Leonardo, 1274 Grens (CH); Ahmed, Shaheen, 8051 Zurich (CH); Goekjian, Peter G., 69006 Lyon (FR); Gueyrard, David, 01120 Montluel (FR); Popowycz, Florence, 69100 Villeurbranne (FR); Schneider, Cedric, 57260 Val de Bride (FR)
(74) Representative: Banfi, Paolo

(57) **Abstract**

Inhibitors of the oncogenic tyrosine kinase ALK and of the Bcr-Abl mutant T315I Bcr-Abl, such as a compound of formula (I): 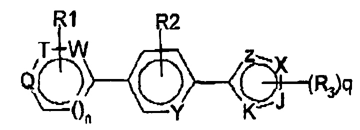 wherein Q, T, W, K, J, Y, X, Z are independently selected from C, N, S, O, provided that the corresponding rings are (hetero)aromatic;
n = 0 or 1;
q = 1 or 2;
pharmaceutical compositions containing the same and their use for the treatment of hyper-proliferative diseases.

## Description

### Field of invention

The present invention provides inhibitors of the oncogenic tyrosine kinase ALK and of the Bcr-Abl mutant T315I Bcr-Abl, pharmaceutical compositions containing the same and their use for the treatment of hyper-proliferative diseases such as cancer, in particular for the treatment of ALK fusion protein positive cancers, such as anaplastic large cell lymphoma (ALCL), diffuse large B cell lymphoma (DLBCL) and inflammatory myofibroblastic tumours (IMT), as well as T315I Bcr-Abl positive cancers such as Chronic Myeloid Leukemia (CML) and Ph+ Acute lymphoblastic leukemia (ALL).

### Background of the invention

Cancer results from the subversion of processes that control the normal growth, location and mortality of cells. This loss of normal control mechanisms arises from the acquisition of mutations that lead to the oncogenic activation of protein kinases. For example, structural alterations in ALK produced by the chromosomal rearrangement t(2q23,5q35) generates the NPM/ALK oncogenic fusion protein associated with ALCL.¹ Whereas, structural alterations in Ab1 produced by the chromosomal rearrangement t(9q34,22q11) generates the Bcr-Abl oncogenic fusion protein associated with CML and ALL.² Within the kinase domain of Bcr-Abl the mutations T315I is critical for the resistance of the tumour towards molecularly targeted therapy.³

Protein kinases are enzymes that catalyse the transfer of phosphate from adenosine-5'-triphosphate (ATP) to specific amino acid residues in many proteins. Generally, the phosphorylation of a protein changes its functionality, from inactive to active in some cases; and from active to inactive in others. Protein kinases are thus involved in the regulation of many aspects of cell function, as most of the signal transduction pathways, such as cellular proliferation, are mediated by phosphorylation. Abnormal activity of protein kinases has been implicated in' many cancers as well as in other diseases. Tyrosine kinases, which phosphorylate the phenolic hydroxyl of tyrosine, are particularly involved in these processes.

Large cell lymphomas represent about 25% of all non-Hodgkin's lymphomas; about one-third of these tumors are anaplastic large cell lymphoma (ALCL). In turn, more than half the patients with ALCL possess a chromosomal translocation that leads to the expression of the NPM/ALK fusion protein. It has been extensively demonstrated that constitutively active NPM/ALK is a potent oncogene with transforming and tumorigenic properties.⁴ The high level of expression of NPM/ALK and other ALK fusion protein variants in lymphoma cells and their direct role in lymphomagenesis, combined with the fact that normal ALK is expressed at low levels in the human body, suggests that ALK could potentially be an ideal target for therapy.

Chronic Myeloid Leukemia (CML) is a myeloproliferative disease, characterized by the presence of a modified chromosome, named Ph-chromosome. In the eighties, the molecular defect associated with this cytogenetic abnormality was identified and it was established that the Ph-chromosome results in the formation of a hybrid gene BCR-ABL coding for the oncogenic fusion protein Bcr-Abl showing tyrosine kinase activity. In the late 1980s, the data accumulated on the role of BCR-ABL in onset and progression of CML indicated BCR-ABL as the most attractive target for molecularly targeted therapy approaches. Therefore attempts to inhibit the TK activity of the oncoprotein were initiated and this process finally ended with the discovery and the development of imatinib mesylate. Imatinib has been under clinical investigation for almost 8 years (50.000 patients) with remarkable results in terms of durable remissions. During the successful clinical trials, resistance to imatinib emerged particularly in patients with acute leukemias, but it is a potential issue also in patients in chronic phase. The molecular mechanism of resistance has been identified in Bcr-Abl gene amplification and mutations in the catalytic kinase domain of the gene³. The mutation of the gatekeeper amino acid threonine into a isoleucine (T315I) has been depicted as the predominant one in patients³. This has prompted intense research to find new compounds able to overcome the resistance problem, such as Dasatinib⁵, SKI-606⁶ and Nilotinib⁷. Despite the increased potency compared to imatinib none of them is able to inhibit efficiently the imatinib-resistant Bcr-Abl T315I mutant. These facts indicate that Bcr-Abl T315I mutant is indeed a target for therapy.

The disclosed inhibition of ALK and Bcr-Abl mutant T315I has been demonstrated using an ELISA-based *in vitro* kinase assay that has been previously developed (EP1454992). Furthermore cellular activity of the compounds on NPM/ALK transformed cells has been demonstrated by tritiated thymidine based cell proliferation inhibition assay.

### Description of the invention

In a first aspect, the invention porvides a compound of formula (I): 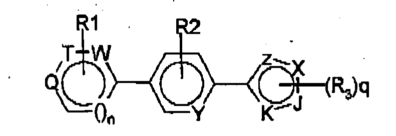 wherein Q, T, W, K, J, Y, X, Z are independently selected from C, N, S, O, provided that the corresponding rings are (hetero)aromatic;
n= 0 or 1;
q=1 or 2;
R1 is selected from: 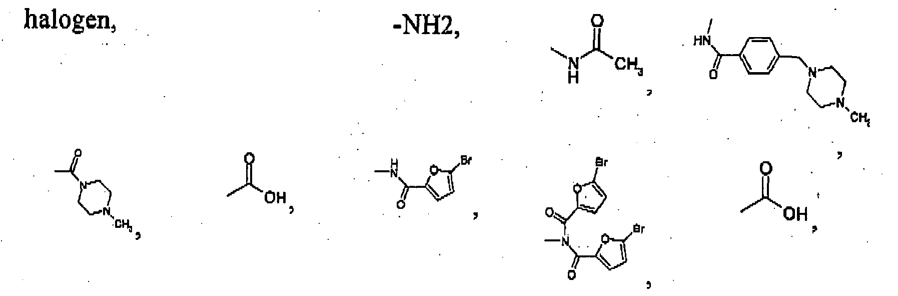 R2 is hydrogen or halogen,
R3 is selected from: 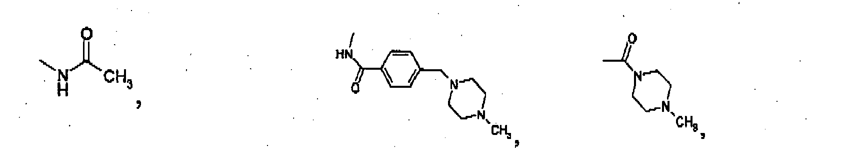 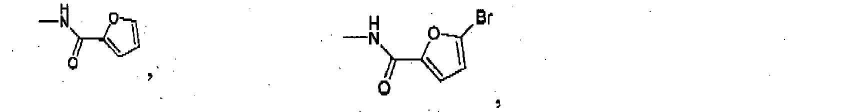 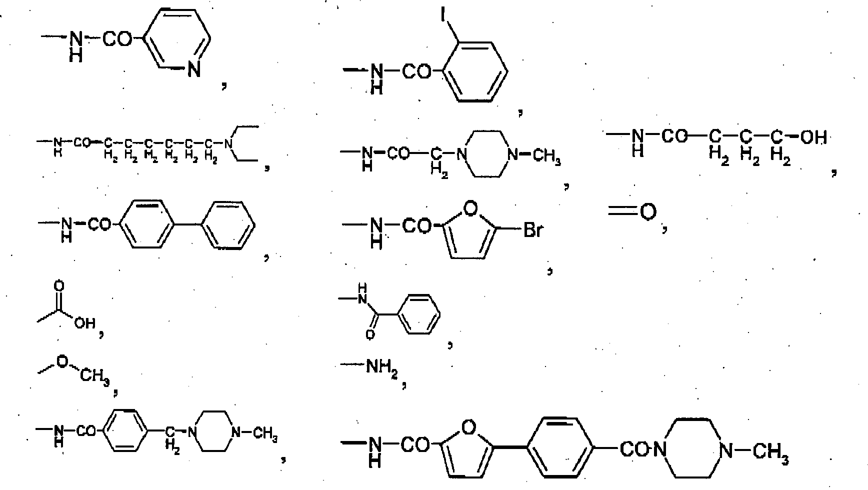 or the moiety 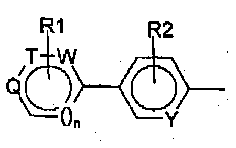 present in formula (I) forms a group 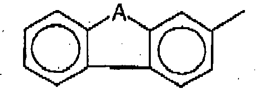 wherein A is -CH2- or -NH-.

In a first preferred embodiment, the invention provides a compound of formula (Ia): 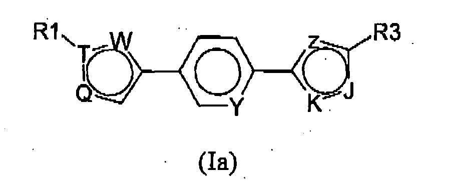 wherein
R1 is selected from 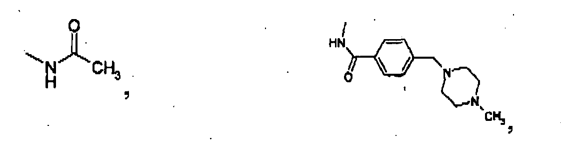 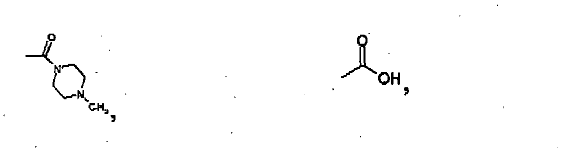 R3 is selected from: 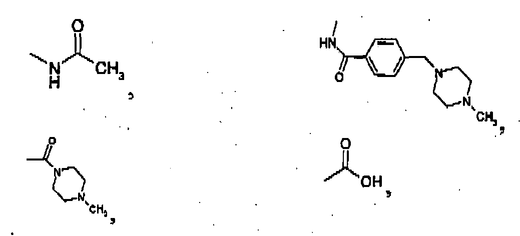 W, T, Q, Y, K, J and Z are as defined above.

In a further preferred embodiment, the invention provides a compound of formula (IIa): 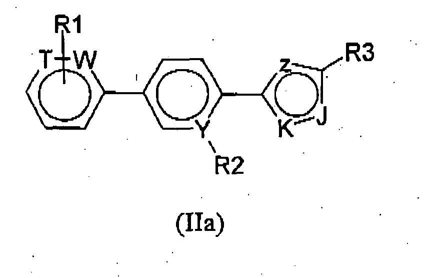 wherein:
R1 is selected from 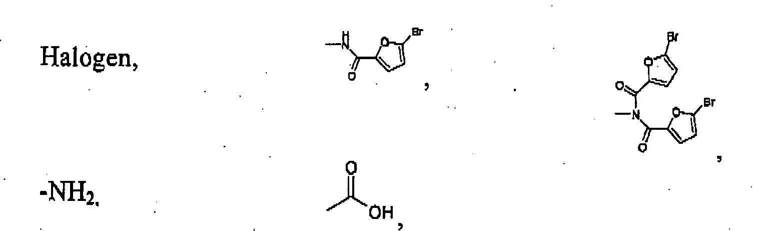
R2 is halogen,
R3 is selected from: 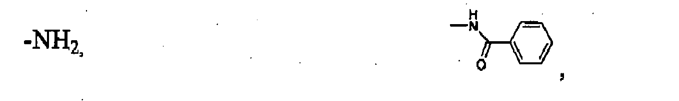 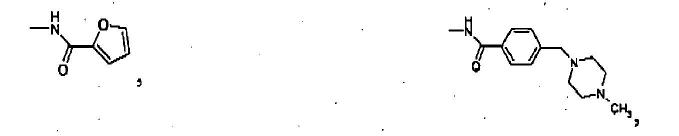 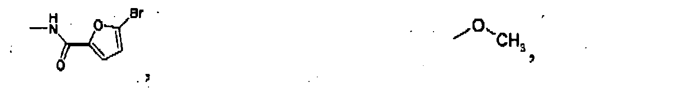
T, W, Y, K, J, Z are as defined above.

In a yet further preferred embodiment, the invention provides a compound of formula (IIIa) 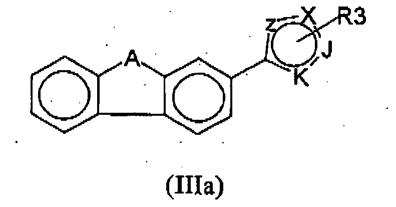 wherein A is -CH2- or -NH-,
R3 is selected from: 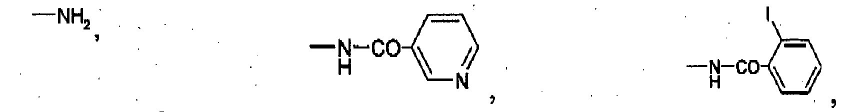 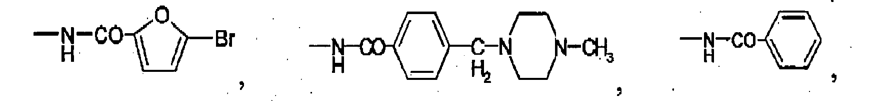 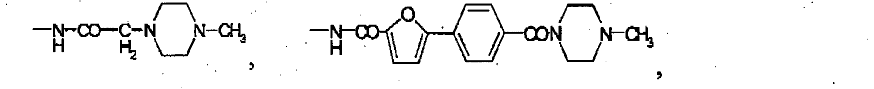 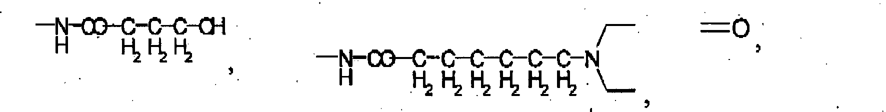 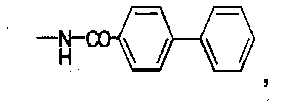 X, Z, J and K are as defined above.

The compounds of the invention can be in the form of free bases or as acid addition salts, preferably salts with pharmaceutically acceptable acids. The invention also includes separated isomers and diastereomers of the compounds, or mixtures thereof (e.g. racemic mixtures).

In a further aspect the invention provides a compound selected from the group consisting of (a) to (n) - the identifier (MDL number) is reported under each compound - for use as a therapeutic agent:

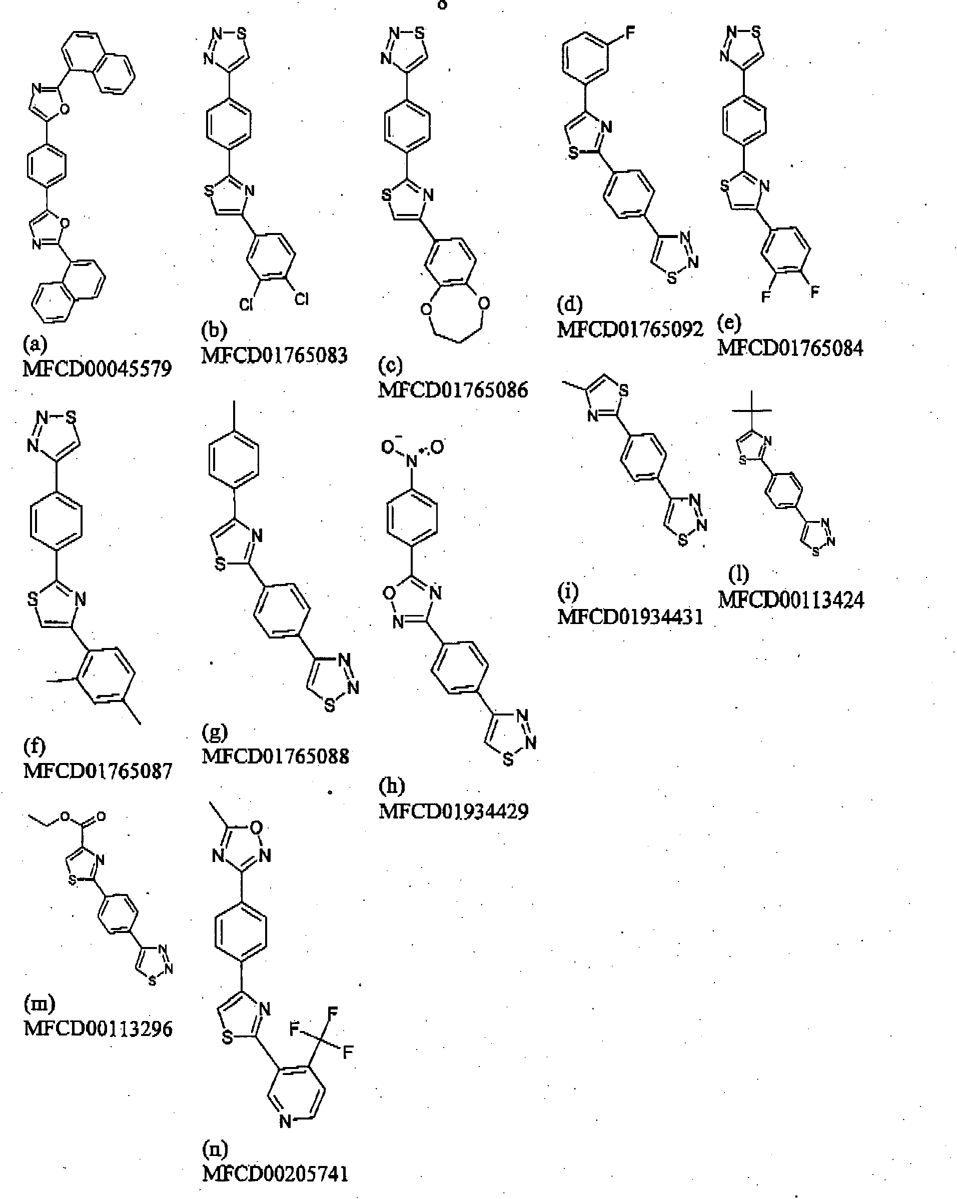

In a yet further embodiment, the invention provides a pharmaceutical composition containing a compound as above described, including compounds (a) to (n), in association with physiologically acceptable carriers and excipients.

The compositions can be in the form of solid, semi-solid or liquid preparations, preferably in form of solutions, suspensions, powders, granules, tablets, capsules, syrups, suppositories, aerosols or controlled delivery systems. The compositions can be administered by a variety of routes, including oral, transdermal, subcutaneous, intravenous, intramuscular, rectal and intranasal, and are preferably formulated in unit dosage form, each dosage containing from about 1 to about 1000 mg, preferably from 1 to 500 mg of active ingredient. The principles and methods for the preparation of pharmaceutical compositions are described for example in Remington's Pharmaceutical Science, Mack Publishing Company, Easton (PA).

In a yet further embodiment, the invention relates to a compound or a pharmaceutical composition as herein provided, including compounds (a) to (n) identified above, for use in the treatment of tumors, especially of Anaplastic Lymphoma Kinase-associated or Bcr-Abl-associated tumors. In a preferred embodiment, the compounds or compositions according to the invention are used in the treatment of anaplastic large cell lymphoma, diffuse large B cell lymphoma, inflammatory myofibroblastic tumors, chronic myeloid leukemia or Ph+ acute lymphoblastic leukemia. In a further preferred embodiment, the compounds or compositions are used for the treatment of chronic myeloid leukaemia (CML) resistant to Imatinib or Dasatinib or Nilotinib or Bosutinib.

### General synthesis strategies

Compounds were designed as a series of three unsaturated rings: two aromatic or heteroaromatic and a terminal five-membered with two heteroatoms. These molecules will be the core for the synthesis of other derivatives; the introduction of a new moiety on the *meta* position on the five membered ring is considered to be promising way to achieve high affinity for the ALK active site.

In order to generate a panel of terminal groups starting from common precursors, the synthetic approach begins from the cross coupling of unsubstituted five-membered rings. In this case the C2 position of the five membered ring is left open to undergo derivatisation after the construction of the polyaromatic scaffold. There are many examples of such reactions, generally based on the different acidity among the proton of the ring: in an 1,3 heteroarmatic structure the C2 position is by far the most acidic, and can be easily and selectively litiated. Amino, alkyl and sulfanyl moieties are examples of the terminal groups that can be attached to the core structure using this approach.

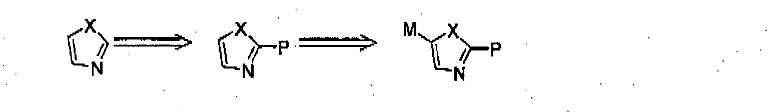

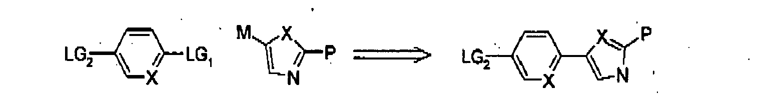

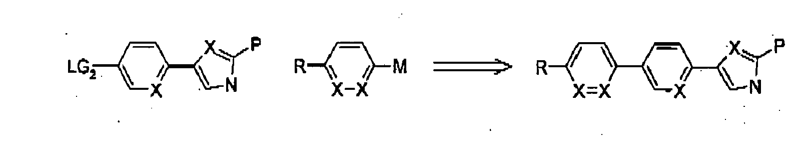

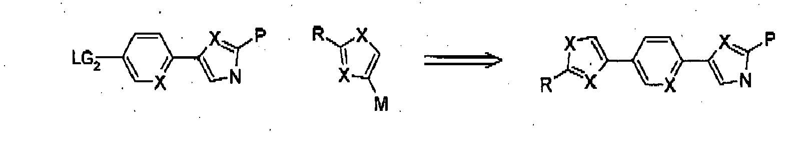

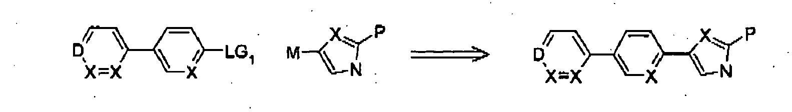

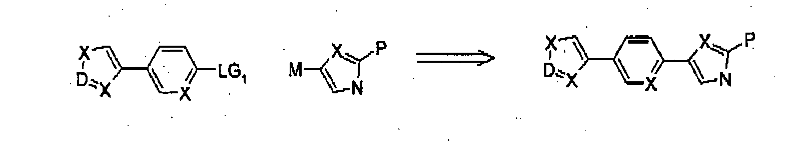

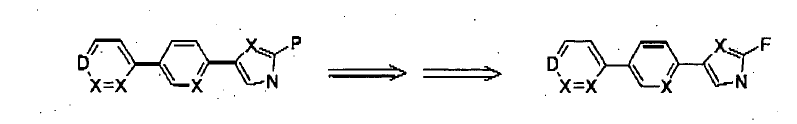

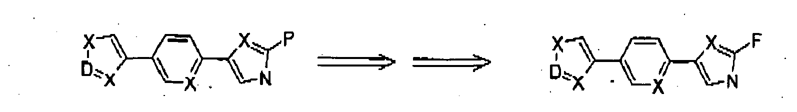

The optimised synthetic path is based on a first Stille coupling between the five-membered metallorganic ring and an aromatic moiety. This has been accomplished using in turn a single aromatic ring with two halogens of different reactivity or a double aromatic ring with a single halogen. In the former case, the second halogen acts as a leaving group in a following carbon-carbon coupling reaction performed using Suzuki or Stille procedure with the appropriate organometallic partner. As already mentioned, the functionalisation of the terminal group is carried out at the end of the synthetic line.

First, the five-membered rings of the terminal moiety were synthesized; the literature methods followed were originally developed by Cliff and Pyne for the stannyl-derivatives of imidazole and by Dondoni for the organometallic thiazoles. The approach towards the functionalization of the C4/C5 position of the two heterocyclic rings is quite different: while for imidazole the metallation happens *via* metal-halogen exchange after halogenation/reductive dehalogenation steps, thiazole can be directly lithiated in C2, protected with trimethylsilyl chloride, and metallated again in C5.

| | |
|---|---|
| 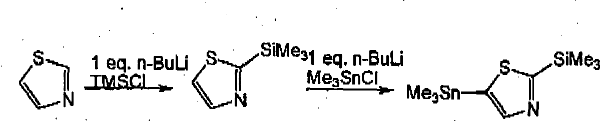 | Dondoni, A.; *et al*.;*J.Org.Chem*., **1988**, 1748-1761 |
| 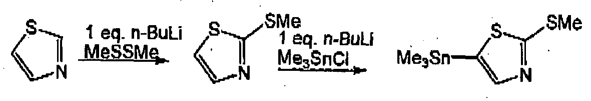 | Dondoni, A.; *et al*.;*J.Org.Chem*., **1988**, 1748-1761 |
| 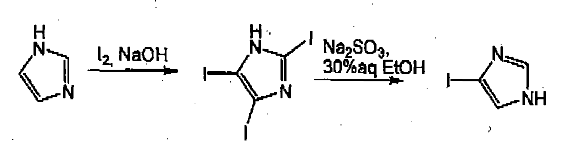 | Cliff, M.D.; Pyne, S.G.; *Synthesis*, **1994**, 681-682 |
| 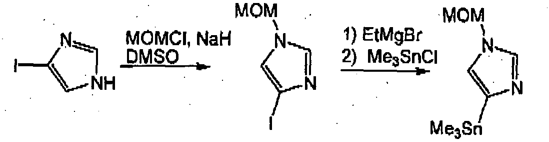 | The use of Grignard in DCM for transmetallation avoids the re-equilibration between C4-C2 positions Ley, S.; *et al*., *J.Org. Chem*., **1991**, 5739-40 |

Once obtained these precursors, their reactivity towards coupling and functionalisation in C2 has been tested. The functionalisation of thiazole with tosyl azide was unreported, but has proven to be feasible. In a test reaction standard addiction of TsN₃ to thiazole after lithiation, followed by hydride reduction of the azide afforded 2-amino thiazole; similarly, also the introduction of a thioether group was quite straightforward:

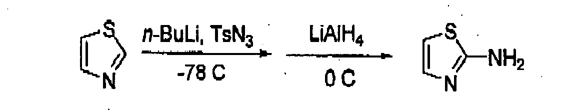

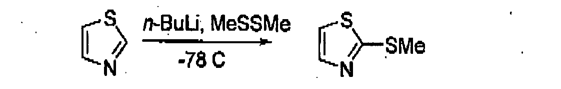

The organo-heterocycles were then used in the Stille coupling. First the adducts of 2-Iodo-5-Chloro pyridine was synthesised, then the 5-Bromine analogue, which was more suitable to undergo a second coupling. Yields were satisfactory for the thiazoles, far worse for the imidazole, probably for the lability of the MOM protecting group under the reaction conditions. 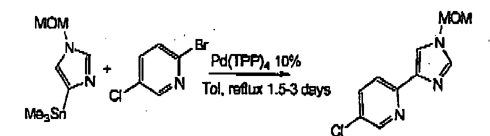 15%-20% yield; 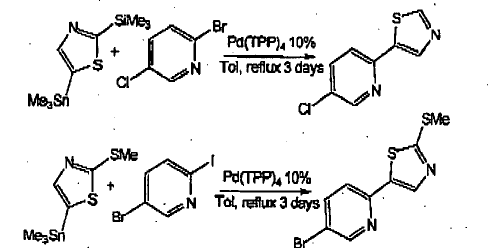 80% yield; silyl moiety was deaved during WU
High yield 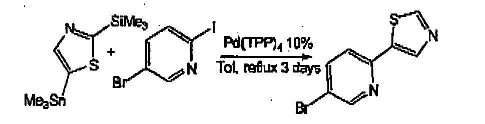 High yield

The regioselectivity of this first Stille reaction is remarkable, as can be expected by the presence of a better leaving group (I vs. Br) on a more electron-poor position, which favours the oxidative insertion of Pd on the carbon-halogen bond. In any case, the α-nitrogen adduct was isolated without trace of other diastereoisomers, as shown by NOE's analysis.

The second addiction could be accomplished via Stille or Suzuki coupling by a tin or boron-aryl compound; we started with 1,2-dichloro-3-methoxybenzene derivatives, which can be synthesised easily both as boronic acid and as stannyl compounds (Perec et al., J. Org. Chem,, 2001, 2104-2117). The following Stille reaction was too slow (no appreciable product formation after 3d), but the Suzuki was satisfactory.

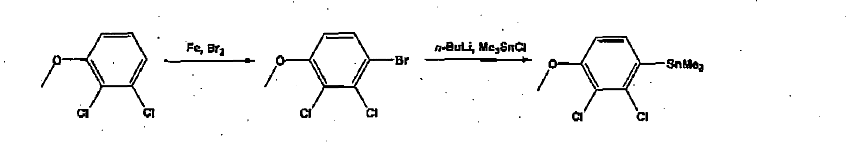

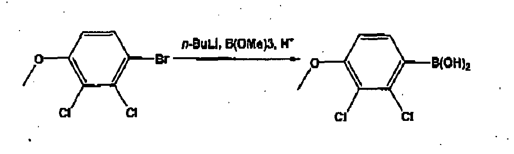

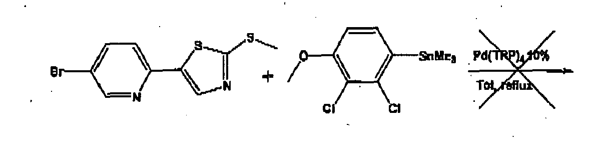

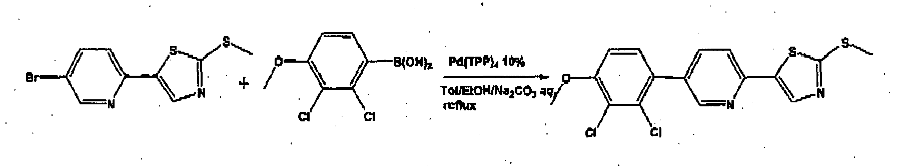

With the same approach, terminal moieties such as the 2-pyridine, 3-pyridine and 2-bromobenzene groups have been introduced coupling them as organo-tin compounds with Stille procedure (2- and 3-pyridine) or as organo-bromic ones with Suzuki (2-bromobenzene, 3-pyridine). Once assessed the reaction conditions, it has been possible to combine the two couplings in a single pot, two step reaction simply adding at the reaction mixture the second organometallic once the first addiction was complete; this allows to spare the use of new catalyst and a chromatographic separation, speeding up considerably the synthetic line.

The derivatization to amide of the terminal amino moiety was studied on 5-(9H-fluoren-7-yl)thiazol-2-amine as a test compound.

Typical coupling procedures were the reaction of acyl chlorides with the amine in solvent mixtures (THF/DMF or DCM/DMF) with triethylamine as base, and reaction with acids assisted by coupling agents such as DCC, HTBU, DIEA and PyBOP with diisopropyl ethylamine or triethylamine as bases. Using these methods the following compounds have been synthesized:
N-(4-(9H-fluoren-2-yl)thiazol-2-yl)-2-(4-methylpiperazin-1-yl)acetamide
N-(4-(9H-fluoren-2-yl)thiazol-2-yl)-7-(diethylamino)heptanamide
N-(4-(9H-fluoren-2-yl)thiazol-2-yl)-4-hydroxybutanamide
N-(4-(9H-fluoren-2-yl)thiazol-2-yl)nicotinamide
N-(4-(9H-fluoren-2-yl)thiazol-2-yl)-5-bromofuran-2-carboxamide
N-(4-(9H-fluoren-2-yl)thiazol-2-yl)-3-(4-methylpiperazin-1-yl)propanamide
5-[4-(4-Methyl-piperazine-1-carbonyl)-phenyl]-furan-2-carboxylic acid [4-(9H-fluoren-2-yl)-thiazol-2-yl]-amide
N-(4-(9H-fluoren-2-yl)thiazol-2-yl)-4-((4-methylpiperazin-1-yl)methyl)benzamide
N-(4-(9H-fluoren-2-yl)thiazol-2-yl)-3,4,5-triiodobenzamide

As already mentioned, the synthesis of tin-substituted thiazoles by literature methods was plagued by low yields; this was mainly due to the low solubility of the anions at low temperature: to obtain the complete formation of the carbanion at -78 C we had to raise the dilution to 1 mmol (85 mg) every 10 ml THF- this procedure is not suitable for large scale preparations, so a one-pot, two steps procedure with sequential addition of the metal chlorides on the C-2 and C-5 carbanions was chosen, raising the temperature after every addiction of BuLi in order to accomplish the formation of the carbanions. In our expectation, the first addition/quench sequence was intended to protect with a TMS group the most acidic C-2 position, while the second one had the aim to introduce the trimethyltin group on the mildly acidic C-5. Through rearrangement the C5-silyl, C2-tin adduct was obtained in really high yield, i.e. the thiazole ring behaved as the C5 position was the most acidic one.

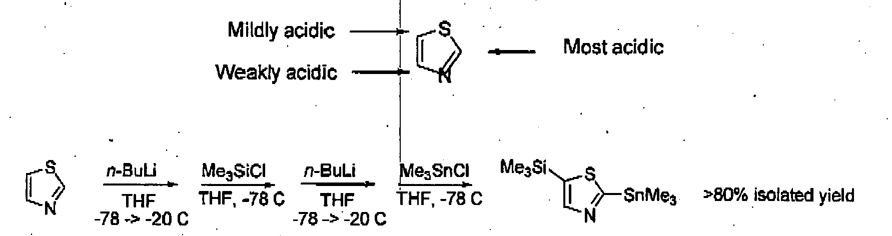

The most straightforward rationale of such behaviour takes into account the different thermodynamic stability of the carbanions: being the conjugate base of the stronger acid, the C2 anion is more stable than the C5 one, and raising the temperature to -20°C is sufficient to have the base rearranged. Reequilibration between the 4 and the 2 position of the imidazole anion has been reported (Groziak, M. P., Wei, L., J. Org. Chem., 1991, 4296-4300).

On the basis of our experimental data, we propose for this rearrangement the mechanism illustrated in Scheme.

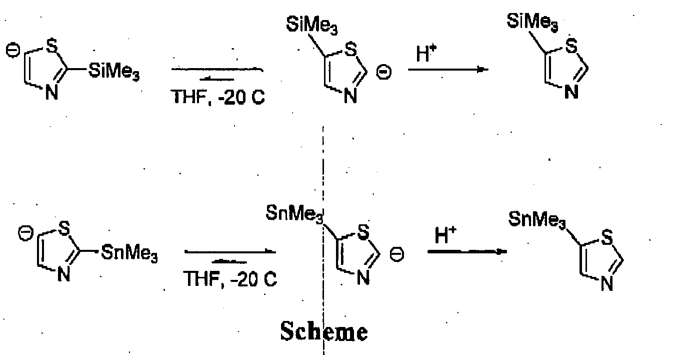

Thus, by controlling the reaction temperature it is possible to functionalise directly the C5 position of the thiazole ring avoiding the use of protection/deprotection procedures.

The rearrangements on 2-trimethyltin and 2-trimethylsilyl thiazole were both effective and afforded the corresponding C5 compounds in high yield.

The same adduct was effective without further purifications in the, above described Stille reactions.

### EXAMPLES

### 1. Synthesis of compounds

### 1) N,N'-(5,5'-(1,4-phenylene)bis(1H-imidazole-5,2-diyl))diacetamide (r114)

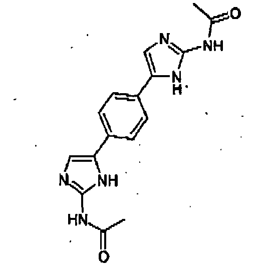

To a solution of acetylguannidine (400 mg, 4mmol) in DMF (5 mL) 1,1'-(1,4-phenylene)bis(2-bromoethanone) (320 mg, 1mmol) was added. The reaction mixture was stirred at RT for 96h, then evaporated, re-taken in water and dried under high vacuum to give 50 mg (15% yield) N,N'-(5,5'-(1,4-phenylene)bis(1H-imidazole-5,2-diyl))diacetamide.
1H-NMR (DMSO, 400 MHz), δ (ppm): 11.72 (bs 1H); 11.28 (bs, 1H); 7.64 (s, 4H); 7.20 (s, 2H); 2.05 (s, 6H).

### 2) 4,4'-(1,4-phenylene)dithiazol-2-amine (precursor of r218)

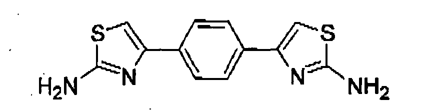

*2-Bromo-1-[4-(2-bromo-acetyl)-phenyl]-ethanone* (0.28 g, 0.9 mmol) was added at room temperature to a stirred solution of thiourea (0.12 g, 1.6 mmol) in hot ethanol (25 mL). The reaction mixture was stirred at 70°C for 3h. After evaporation of the solvent under reduced pressure, the crude was purified by flash chromatography (94:5:1, CHCl₃:EtOH:Et₃N) giving 190 mg (77%) of *4,4'-(1,4-phenylene)dithiazol-2-amine*.
¹H NMR (DMSO, 400 MHz), δ (ppm): 7.80 (s, 4H), 7.19 (s, 2H), 3.51 (bs, NH₂).

### 3) N,N'-(4,4'-(1,4-phenylene)bis(thiazole-4,2-diyl))bis(4-((4-methylpiperazin-1-yl)methyl)benzamide) (r218)

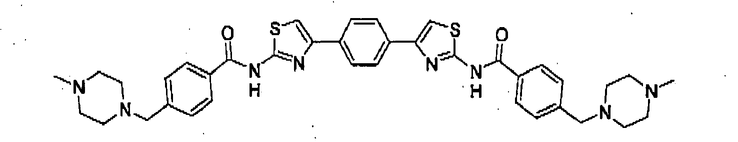

*4-(4-Methyl-piperazin-1-ylmethyl)-benzoic acid* (0.38 g; 1.6 mmol) was dissolved in CH₂Cl₂ (20 mL), and *N,N-diisopropylethylamine* (0.30 mL, 2.0 mmol). After 10 min, HBTU (0.26 g, 0.7 mmol) and *4,4'-(1,4-phenylene)dithiazol-2-amine* (0.19 g, 0.7 mmol) were added. The reaction was stirred at room temperature overnight. After evaporation of the solvent under reduced pressure, the crude was purified by flash chromatography (94:5:1 CH₂Cl₂:EtOH:Et₃N) giving 200 mg (40%) of *N,N'-(4,4'-1,4-phenylene)bis(thiazole-4,2-diyl))bis(4-4((4-methylpiperazin-l-yl)methyl)benzamide*
¹HNMR (DMSO, 400 MHz), δ (ppm): 8.00 (d, 4H, *J* =8.2 Hz), 8.02 (s, 4H), 7.73 (s, 2H), 7.48 (d, 4H, *J* = 8.2 Hz), 3.59 (s, 4H), 2.50 (bs, 16H), 2.45 (s, 6H)

### 4) 1,4-di(furan-3-yl)benzene (r236)

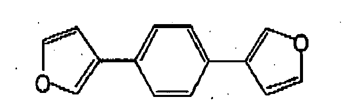

Palladium tetrakistryphenilphosphine (0.20 mmol, 0.24 g) was added to a solution of 1,4-dibromobenzene (1.0 g, 4.4 mmol), furan-3-yl boronic acid (0.40 g, 3.6 mmol) and cesium carbonate (4.2 g, 13 mmol) in dimethoxyethane/water (15/5 mL), and the resulting mixture degassed and refluxed under argon for 14h.

The resulting suspension was cooled, filtered and concentrated under reduced pressure. The crude product was purified by flash chromatography (95: 5 hexane ethyl acetate) to give 100 mg (13%) of *1,4-di(furan-3-yl)benzene* as a white solid.
¹H NMR (400 MHz, CDCl₃) δ (ppm): 7.75 (dd, 2H, J=1.0 Hz, J=1.5 Hz) 7.49 (m, 6H) 6.72 (dd, 2H, J=0.9 Hz, J=1.9 Hz).

### 5) 5-Bromo-furan-2-yl)-(4-methyl-piperazin-1-yl)-methanone

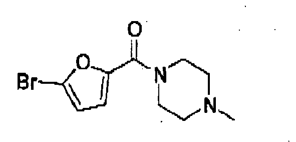

*5-Bromo-furan-2-carboxylic acid* (2.0 g, '10 mmol) was suspended in thionyl chloride (10 mL). The reaction mixture was heated to 100°C, two drops of DMF were added, and the resulting solution was refluxed for 1h. After cooling, thionyl chloride was removed under reduced pressure, and the residue was re-taken in Et3N (3 mL) and anhydrous THF (25 mL). The solution was filtered and 1-methyl-piperazine (1.55 mL, 14 mmol) was added. The reaction mixture was heated to 70°C overnight After evaporation of the solvent under reduced pressure, the crude was purified by flash chromatography (97:2:1 CHCl₃:EtOH:Et₃N) giving 2.20 g (80%) of *(5-Bromo-furan-2-yl)-(4-methyl-piperazin-1-yl)-methanone*.
¹HNMR (CDCl₃, 400 MHz), δ (ppm): 6.98 (d, 1H, *J* =3.6 Hz), 6.42 (d, 1H, *J* =3.6 Hz), 3.83 (bs, 4H), 2.36 (s, 3H), 1.80 (bs, 4H).

### 6) (5-{4-[5-(4-Methyl-piperazine-1-carbonyl)-furan-2-yl]-phenyl}-furan-2-yl)-(4-methyl-piperazin-1-yl)- methanone (r237)

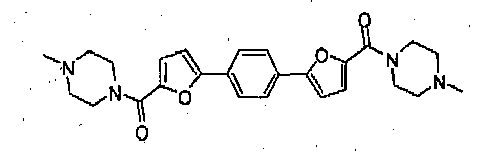

Palladium tetrakistryphenilphosphine (40 mg, 36 µmol) was added to a stirred solution of *(5-Bromo-furan-2-yl)-(4-methyl-piperazin-1-yl)-methanone* (0.40 g, 1.4 mmol) and *1,4-phenylene diboronic acid* (0.12 g, 0.7 mmol) in degassed dioxane (15 mL) and saturated aqueous sodium carbonate (8 mL). The reaction mixture was refluxed overnight under Argon atmosphere. After evaporation of the solvent, the crude was purified by flash chromatography (89:10:1 CHCl₃: EtOH: Et₃N,) giving 260 mg (80%) of *(5-{4-[5-(4-Methyl-piperazine-1-carbonyl)-furan-2-yl]-phenyl}-furan-2-yl)-(4-methyl-piperazin-1-yl)- methanone*.
¹H NMR (CDCl₃, 400 MHz), δ (ppm): 7.70 (s, 4H), 7.26 (d, 2H, *J* =3.6 Hz), 6.78 (d, 2H, *J* =3.6 Hz), 3.94 (bs, 8H), 2.40 (s, 6H), 1.85 (bs, 8H).

### 7) 5,5'-(1,4-phenylene)difuran-2-carboxylic acid. (r239)

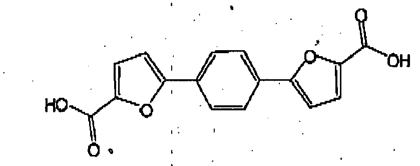

Palladium tetrakistryphenilphosphine (0.10 mmol 0.30 g) was added to a solution of 1,4-phenylenediboronic acid (0.40 g, 2.5 mmol), cesium carbonate (3.20 g, 10 mmol) and 5-bromofuran-2-carboxylic acid (0.95 g, 5 mmol) in dimethoxyethane/water (10/5 mL); the resulting mixture was degassed and refluxed under argon. After 24 h the solvent was evaporated *in vacuo* and the crude product purified by flash chromatography (98.2 CH₂Cl₂ CH₃OH) to give 0.15 g (20%) of *5,5'-(1,4-phenylene)difuran-2-carboxylic acid* (0.15 g, 0.5 mmol) as a white solid.
¹H NMR (d6-DMSO 400 MHz) δ (ppm): 7.85 (m, 4H) 7.20 (m, 2H) 7.16 (d, 2H, J=3.5 Hz).

### 8) 5-chloro-2-(1-(methoxymethyl)-1H-imidazol-4-yl)pyridine

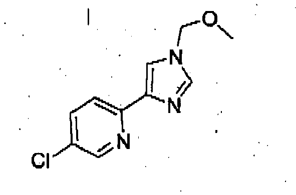

Pd(PPh₃)₄ (110 mg, 0.1 mmol) was added portiowise to a stirred solution of 1-(methoxymethyl)-4-(trimethylstannyl)-1H-imidazole (550 mg, 2 mmol) and 2-bromo-5-chloropyridine (400 mg, 2.2 mmol) in degassed toluene (20 ml) under inert atmosphere. The reaction mixture was refluxed for 2d. After evaporation of the solvent under reduced pressure, the crude was purified by flash chromatography (hexane/AcOEt, 70:30) giving 70 mg (15%) of 5-chloro-2-(1-(methoxymethyl)-1H-imidazol-4-yl)pyridine.
1H NMR (400 MHz, CDCl3), δ (ppm):

### 9) 5-chloro-2-(thiazol-5-yl)pyridine

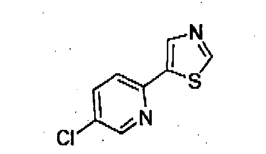

Pd(PPh₃)4 (110 mg, 0.1 mmol) was added portiowise to a stirred solution of 2-(trimethylsilyl)-5-(trimethylstannyl)thiazole (320 mg, 1 mol) and 2-bromo-5-chloropyridine (200 mg, 1.1 mmol) in degassed toluene (10 ml), under inert atmosphere. The reaction mixture was refluxed for 1 d. After evaporation of the solvent under reduced pressure, the crude was purified by flash chromatography (hexane/AcOEt 70:30%). Deprotection of the silyl group occurs during chromatography give 150 mg (80%) of 5-chloro-2-(thiazol-5-yl)pyridine.
1H NMR (400 MHz, CDCl3), δ (ppm): 8,48 (1 H, d); 8,32 (1 Hm s); 7,95 (1 H, s); 7,72 (1 H, d); 7,69 (1H, 1 H, s)

### 10) 5-bromo-2-(thiazol-2-yl)pyridine

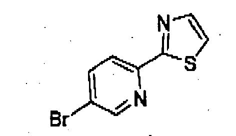

Pd(PPh₃)₄ (220 mg, 0.2 mmol) was added portionwise to a stirred solution of 2-(trimethylstannyl)thiazole (0.50 g, 2mmol) and 2-Iodo-5-Bromopyridine (0.57 g, 2 mmol) in degassed toluene (20 ml) under inert atmosphere. The reaction mixture was refluxed for 14 h. After evaporation of the solvent under reduced pressure, the crude was purified by flash chromatography (hexane/AcOEt, 80:20) giving 400 mg (83%) of 5-bromo-2-(thiazol-2-yl)pyridine.
1H NMR (400 MHz, CDC13), δ (ppm):

### 11) 5-bromo-2-(thiazol-5-yl)pyridine (precursor r113)

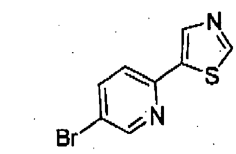

Pd(PPh₃)₄ (220 mg, 0.2 mmol) was added portionwise to a stirred solution of 2-(trimethylsilyl)-5-(trimethylstannyl)thiazole (640 mg, 2 mmol) and 2-Iodo-5-Bromopyridine (0.57 g, 2 mmol) in degassed toluene (20 ml), under inert atmosphere. The reaction was refluxed for 14 h and quenched with with saturated Na₂CO₃. After quenching with saturated Na₂CO₃ solution, the mixture was extract with ether. The organic layers were dried with MgSO₄ and the solvent removed under reduced pressure. The crude was purified by flash chromatography (hexane/AcOEt, 70:30) giving 330 mg (70%) of 5-bromo-2-(thiazol-5-yl)pyridine.
¹H NMR (400 MHz, CDC13), δ (ppm): 8,84 (1 H, s); 8,54 (1 H, d); 8,32 (1 H, s); 7,70 (1 H, dd); 7,63 (1 H, d);

### 12) 5-(2-bromophenyl)-2-(thiazol-2-yl)pyridine (r116)

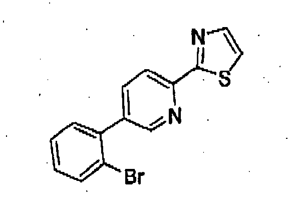

Pd(PPh₃)₄ (10 mg, 0.01 mmol) was added portiowise to a stirred solution of 2-bromophenylboronic acid (100 mg, 0.5 mmol) and 5-bromo-2-(thiazol-5-yl)pyridine (100 mg, 0.4 mmol) in dioxane (5 ml) under inert atmosphere. A solution of K₂CO₃ (200 mg, 1.5 mmol) in water (2 ml) was then added and the reaction refluxed for 14 h. After quenching with saturated Na₂CO₃ solution, the mixture was extract with ether. The organic layers were dried with MgSO₄ and the solvent removed under reduced pressure. The crude was purified by flash chromatography (hexane/AcOEt, 30:70) giving 60 mg (50%) of 5-(2-bromophenyl)-2-(thiazol-2-yl)pyridine.
¹H NMR (400 MHz, CDCl3), δ (ppm): 8,68 (1 H, d); 8,26 (1 H, dd); 7,95 (1 H, d); 7,89 (1 H, dd); 7,72 (1 H, dd); 7,48 (1 H, d); 7,43 (1 H, td); 7,37 (1 H, dd); 7,29 (1 H, td)

### 13) 2-(6-(thiazol-5-yl)pyridin-3-yl)pyridine (r117)

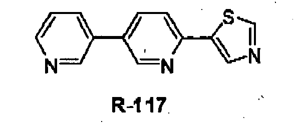

3-(trimethylstannyl)pyridine (0.24 g, 0.9 mmol) dissolved in anhydrous toluene (5 mL) was added dropwise to a solution of 5-(5-bromo pyridin-2-yl)thiazole (0.12 g, 0.5 mmol) in (10 mL) under Argon atmosphere; the reaction is stirred at reflux for 14 h. The solvent was then removed under vacuum and the crude purified by column chromatography (eluant: CH₂Cl₂/MeOH, 99:1) to give 0.06 g, 0.24 mmol of 2-(6-(thiazol-5-yl)pyridin-3-yl)pyridine (60% yield).
¹H NMR (CDCl₃ 400 MHz) δ (ppm):8.88 (s, 2H), 8.84 (d, 1H, J=2.3 Hz), 8.68 (d, 1H, J=4.8 Hz), 8.41 (s, 1H), 7.96 (dd, 1H, J1=8.1, J2=2.3 Hz), 7.92 (dt, 1H, J1=1.3, J2=7.9 Hz), 7.83 (d, 1H, J=8.1 Hz), 7.45 (dd, 1H, J1=8.1, J2=4.8 Hz).

### 14) 3-(3-Chloro-4-thiazol-5-yl-phenyl)-pyridine (r120)

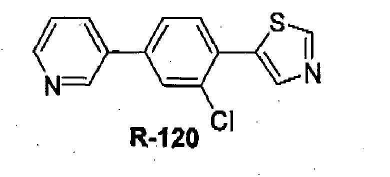

To a solution of *5-(4-Bromo-2-chloro-phenyl)-thiazole* (3.4 g, 12 mmol) and *3-[1,3,2]Dioxaborinan-2-yl-pyridine* (2.0 g, 12 mmol) in 15 mL of toluene in a Schlenk apparatus 5 mL of a saturated solution of Na₂CO₃ in water were added. The mixture was degassed and [Pd(P(Ph₃)₄] (630 mg, 60 mmol) was added and the reaction stirred at 110 C for 12 h. The solvent is then evaported and the crude re-taken in CHCl₃ and filtered on celite and purified by column chromatography (eluant: 15:85, CHCl₃/AcOEt) to give 1.40 g (42% yield) of 3-(3-Chloro-4-thiazol-5-yl-phenyl)-pyridine as a yellow solid.

¹H NMR (CDCl₃ 400 MHz), δ (ppm): 8.90 (s, 1H), 8.87 (d, 1H, *J* = 2.0), 8.65 (dd, 1H, *J* = 4.7, 2.0 Hz), 8.16 (s, 1H), 7.90 (m, 1H), 7.73 (d, 1H, *J* = 1.6 Hz), 7.65 (d, 1H, *J* = 8.2 Hz), 7.54 (dd, 1H, *J* = 8.2, 2.0 Hz), 7.41 (m, 1H).

### 15) N-[5-(2-Chloro-4-pyridin-3-yl-phenyl)-thiazol-2-yl]-4-(4-methylpiperazin-1-ylmethyl)-benzamide (r127)

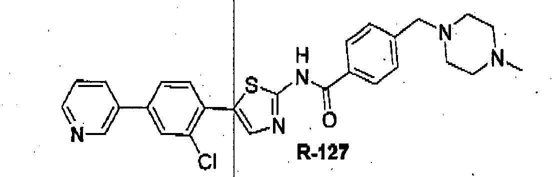

To a solution of *4-(4-Methyl-piperazin-1-ylmethyl)-benzoic acid* (122 mg, 0.5 mmol) in DMF (15 mL) and DIEA (0.2 mL, 1.1 mmol) HBTU (200 mg, 0.5 mmol) e *5-(2-Chloro-4-pyridin-3-yl-phenyl)-thiazol-2-ylamine* (150 mg, 0.5 mmol) were added. The reaction was stirred at RT for 12h, then the solvent was removed under reduced pressure and the crude purified by column chromatography (eluant: 99:1, CHCl₃/Et₃N). Obtained 176 mg (70% yield) of N-[5-(2-Chloro-4-pyridin-3-yl-phenyl)-thiazol-2-yl]-4-(4-methyl-piperazin-1-ylmethyl)-benzamide.
¹H NMR (DMSO, 400 MHz), δ (ppm): 8.86 (dd, 1H, *J* = 2.4, 0.8 Hz), 8.65 (dd, 1H, *J* = 4.8, 1.6 Hz), 7.99 (m, 2H), 7.89 (ddd, 1H, *J* = 7.9, 2.4, 1.6 Hz), 7.71 (d, 1H, *J* = 1.9 Hz), 7.61 (d, 1H, *J* = 8.1 Hz), 7.56 (s, 1H), 7.53 (m, 2H), 7.52 (dd, 1H, *J* = 8.0, 2.0 Hz), 7.41 (ddd, 1H, *J* = 8.0, 4.9, 1.0 Hz), 3.6 (s, 2H), 2.50 (s largo, 4H), 2.46 (s largo, 4H), 2.28 (s, 3H)

### 16) 5-Bromo-furan-2-carboxylic acid [5-(2-chloro-4-pyridin-3-yl-phenyl)-thiazol-2-yl] -amide (r128)

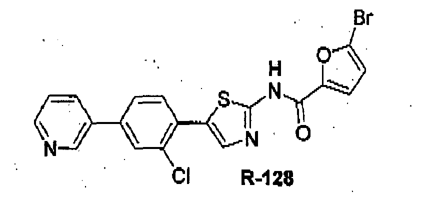

*5-(2-Chloro-4-pyridin-3-yl-phenyl)-thiazol-2-ylamine* (150 mg, 0.5 mmol) and HTBU were added to a solution of *5-Bromo-furan-2-carboxylic acid* (100 mg, 0.5 mmol) in DMF (15 mL) and DIEA (0.2 mL, 1.1 mmol). The reaction was stirred at RT for 12 h, the solvent was then removed under reduced pressure and the crude purified by column chromatography (eluant: 98:1:1, CHCl₃/EtOH/Et₃N) to give 98 mg (40% yield) of 5-Bromo-furan-2-carboxylic acid [5-(2-chloro-4-pyridin-3-yl-phenyl)-thiazol - 2-yl] -amide.
¹H NMR (DMSO, 400 MHz), δ (ppm): 9.90 (bs, 1H), 8.97 (d, 1H, *J* = 2.4 Hz), 8.69 (dd, 1H, *J* = 4.8, 1.7 Hz), 8.17 (dt, 1H, *J* = 8.2, 2.2 Hz), 7.98 (m, 1H), 7.93 (s, 1H), 7.79 (s, 2H), 7.54 (m, 1H), 7.51 (dd, 1H, *J* = 8.0, 4.8 Hz), 6.84 (d, 1H, *J* = 3.6 Hz).

### 17) 5-bromo-N-(5-(6-(oxazol-2-yl)pyridin-3-yl)pyridin-2-yl)furan-2-carboxamide (r200)

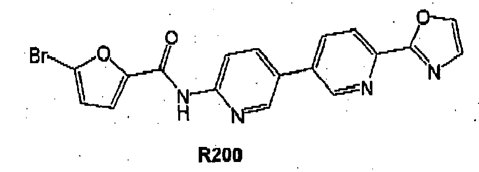

To a solution of 5-bromofuran-2-carbonyl chloride (18.0 g, 0.9 mmol) in dry DCM (10 ml) and freshly distilled Et₃N (0.4 ml) 5-(6-(oxazol-2-yl)pyridin-3-yl)pyridin-2-amine (0.15 g, 0.6 mmol). The mixture is stirred at RT under Argon atmosphere for 1h, evaporated and purified by by column chromatography (eluant: 60:35:5 CHCl3:EtOAc:Et3N). Obtained 0.04 g (17% yield) of 5-bromo-N-(5-(6-(oxazol-2-yl)pyridin-3-yl)pyridin-2-yl)furan-2-carboxamide.
¹H NMR (CDCl₃ 400 MHz) δ(ppm):8.96 (d, 1H, *J*=2.2Hz), 8.62 (d, 1H, *J*=2.4Hz), 8.49 (d, 1H; *J*=8.6Hz), 8.26 (d, 1H, *J*=8.2Hz), 8.04 (dd, 2H, *J*=2.3Hz, *J*=8.2Hz), 7.86 (s, 1H), 7.36 (s, 1H), 7.29 (d, 1H, *J*=3.6Hz), 6.55 (d, 1H, *J*=3.6Hz).

### 18) 4-(4-bromo-3-chlorophenyl)-2-methoxythiazole

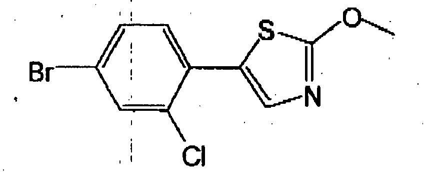

A solution of sodium methoxide in dry methanol under Argon atmosphere was cooled to 0°C and 4-(4-bromo-3-chlorophenyl)thiazole (0.2 g, 0.6 mmol) in dry methanol (5 mL) was added dropwise. The reaction mixture is warmed to RT and refluxed for 4h, then cooled, diluted with water (20 mL) and extracted with ether (3x20 mL). The organic phases are dried (MgSO₄) and evaporated to give 0.15 g (84% yield) of 4-(4-bromo-3-chlorophenyl)-2-methoxythiazole.
¹H NMR (CDCl₃ 400 MHz) δ(ppm):7.62 (d, 1H, *J*=1.9 Hz), 7.41 (dd, 1H, *J*=2.0 Hz, *J*=8.3 Hz), 7.34 (s, 1H), 7.31 (d, 1H, *J*=8.3 Hz), 4.11 (s, 3H).

### General procedure for compounds r238, r235 r262

To a 0.1 M solution of boronic acid (1 eq) in Dioxane:H2O 5:1 the organic halide (1.2 eq) and K2CO3 (5 eq) are added. The solution is degassed, [PdP(Ph3)4] (5% mol) is added and reaction is heated to reflux and monitored by TLC. Upon completion, the solvent is evaporated and the crude re-taken in MeOH or Chloroform, filtered and purified by column chromatography.

### 19) 2-(4-bromo-2-chlorophenyl)furan

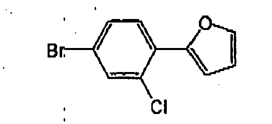

Obtained by reaction of 4-bromo-2-chloro-1-iodobenzene with furan-2-yl-2-boronic acid. Yield after purification by column chromatography (eluant:hexane): 30%.
¹H NMR (CDCl₃ 400 MHz) δ(ppm):7.73 (d, 2H, *J*=8.6 Hz), 7.60 (d, 1H, *J*=2.0 Hz), 7.52 (dd, 1H, *J*=0.6 Hz, *J*=1.8 Hz), 7.44 (dd, 1H, *J*=2.0 Hz, *J*=8.5 Hz), 7.14 (dd, 1H, *J*=0.5 Hz, *J*=3.5 Hz), 6.53 (dd, 1H, *J*=1.8 Hz, *J*=3.4 Hz).
¹³C NMR (CDCl3 100 MHz) δ(ppm): 149.33, 142.37, 133.15, 132.02, 131.20, 130.14, 128.80, 120.70, 111.85, 111,33.

### 20) 5-(2-cloro-4-(piridin-3-il)fenil)-2-metossitiazolo (r262)

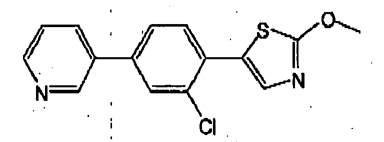

Obtained by reaction of 4-(4-bromo-3-chlorophenyl)-2-methoxythiazole with pyridin-3-ylboronic acid. Yield after purification by column chromatography (eluant:hexane:ethyl acetate 6:4): 30%
¹H NMR (CDCl₃ 400 MHz) δ(ppm): 8.86 (s, 1H), 8.64 (d, 1H, *J*=4.2 Hz), 7.89 (dt, 1H, *J*=7.9 Hz, *J*=1,9 Hz), 7.69 (d, 1H, *J*=1.8 Hz), 7.58 (d, 1H, *J*=8.1 Hz), 7.50 (dd, 1H, *J*=1.8 Hz, *J*=8.1 Hz), 7.42 (m, 2H), 4.13 (s, 3H).
¹³C NMR (CDCl3 100 MHz) δ(ppm): 140.08, 136.53, 134.47, 134.26, 134.19, 131.32 (2C), 128.94, 125.61 (2C), 123.64, 58.17.

### 21) 3'-cloro-4'-(furan-2-il)bifenil-4-acido carbossilico (r238)

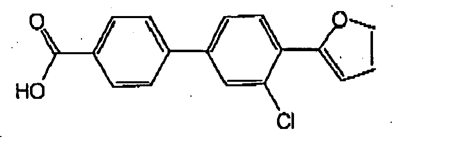

Obtained by reaction of 2-(4-bromo-2-chlorophenyl)furan with 4-boronobenzoic acid. Yield after purification by column chromatography (eluant: CHCl3: methanol 95:5): 30%.
¹H NMR (d6-DMSO 400 MHz) δ(ppm):8.01 (d, 2H, *J*=8.3 Hz), 7.93 (dd, 2H, *J*=3.2 Hz, *J*=5.1 Hz), 7.87 (s, 2H), 7.85 (s, 1H), 7.81 (dd, 1H, *J*=1.8 Hz, *J*=8.4 Hz), 7.20 (d, 1H, *J*=3.5 Hz), 6.68 (dd, 1H, *J*=1.8 Hz, *J*=3.3 Hz).
¹³C NMR (d6-DMSO 100 MHz) δ(ppm): 143.66, 143.63, 130.00, 129.65, 128.84, 128.30, 128.02, 126.76, 126.00, 122.21, 111.53.

### 22) 5-(3-cloro-4-(tiazol-5-il)fenil)piridin-2-ammina (r235)

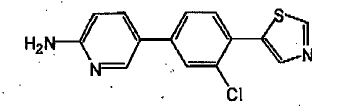

Obtaine by reaction of 4-(4-bromo-3-chlorophenyl)thiazole with 6-aminopyridin-3-ylboronic acid. Yield after purification by column chromatography (eluant: DCM: methanol 95:5): 42%.
¹H NMR (CDCl₃ 400 MHz) δ(ppm): 8.88 (d, 1H, J=0.6Hz) 8.30 (dd, 1H, *J*=0.6 Hz, *J*=2.4 Hz), 8.14 (d, 1H, *J*=0.7 Hz), 7.73 (dd, 1H, *J*=2.5 Hz, J=8.7 Hz), 7.64 (d, 1H, *J*=1.8 Hz), 7.59 (d, 1H, *J*=8.1 Hz), 7.45 (dd, 1H, *J*=1.9 Hz, *J*=8.1 Hz), 6.68 (dd, 1H, *J*=0.7 Hz, *J*=8.6 Hz), 5.04 (s, 2H).

### 23) 5-(2,3-dichloro-4-methoxyphenyl)-2-(2-(methylthio)thiazol-5-yl)pyridine

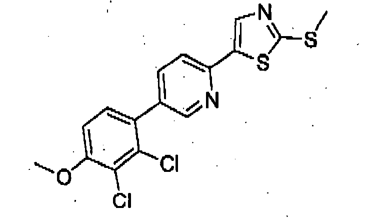

Pd(PPh₃)₄ (100 mg, 0.1 mmol) was added portiowise to a stirred solution of 2,3-dichloro-4-methoxyphenylboronic acid (220 mg, 1.0 mmol) and 5-bromo-2-(2-(methylthio)thiazol-5-yl)pyridine (350 mg, 1.0 mmol) in dioxane (5 ml) under inert atmosphere. A solution of K₂CO₃ (1 g, 7.2 mmol) in water (10 ml) was then added and the reaction refluxed for 14 h. After quenching with saturated Na₂CO₃ solution, the mixture was extract with ether. The organic layers were dried with MgSO₄ and the solvent removed under reduced pressure. The crude was purified by flash chromatography (hexane/AcOEt, 50:50) giving 110 mg (30%) of 5-(2,3-dichloro-4-methoxyphenyl)-2-(2-(methylthio)thiazol-5-yl)pyridine.
¹H NMR (400 MHz, CDCl₃), δ (ppm): 8,57 (1H, d); 8,12 (1H, s); 7,77 (1 H, dd); 7,66 (1H, d); 7,22 (1H, d); 6,97 (1 H, d); 3,97 ( 3 H, s); 2,75 (3 H, s)

### 24) 5-(2,3-dichloro-4-methoxyphenyl)-2-(thiazol-5-yl)pyridine

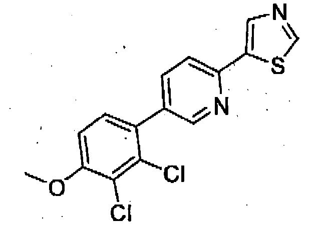

Pd(PPh₃)₄ (100 mg, 0.1 mmol) was added portionwise to a stirred solution of 2,3-dichloro-4-methoxyphenylboronic acid (220 mg, 1.0 mmol) and 5-bromo-2-(thiazol-5-yl)pyridine (240 mg, 1.0 mmol) in dioxane (5 ml) under inert atmosphere. A solution of K₂CO₃ (1 g, 7.2 mmol) in water (10 ml) was then added, and the reaction was refluxed for 14 h. After quenching with saturated Na₂CO₃ solution, the mixture was extract with ether. The organic layers were dried with MgSO₄ and the solvent removed under reduced pressure. The crude was purified by flash chromatography (hexane/AcOEt 50:50) giving 140 mg (30%) of 5-(2,3-dichloro-4-methoxyphenyl)-2-(2-(methylthio)thiazol-5-yl)pyridine.
¹H NMR (400 MHz, CDCl₃), δ (ppm): 8,90 (1H, s); 8,63(1 H, d); 8,45 (1 H, s); 7,84 (1H dd); 7,79 (1 H, d); 7,23 (1 H, d); 6,99 (1H, d); 3,98 (3H, s)

### 25) 3-(6-(thiazol-2-yl)pyridin-3-yl)pyridine

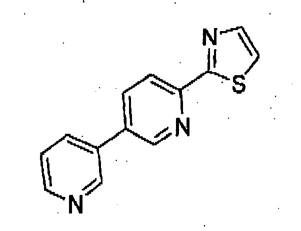

Pd(PPh₃)₄ (100 mg, 0.1 mmol) was added portiowise to a stirred solution of 5-bromo-2-(thiazol-2-yl)pyridine (480 mg, 2.0 mmol) in dry toluene (20 ml) under inert atmosphere, followed by addition of pyridin-3-yl-3-boronic acid (280 mg, 2.3 mmol) in EtOH (5 ml). A 2 N aqueous solution of Na₂CO₃ (2.5 ml, 5 mmol) was then added and the reaction refluxed for 12 h. The solution was diluted with ether washed with saturated Na₂CO₃ solution, and the organic layers dried with MgSO₄. The solvent was removed under reduced pressure. The crude was purified by flash chromatography (hexane/AcOEt 90:10), giving 300 mg (60%) of 3-(6-(thiazol-2-yl)pyridin-3-yl)pyridine.
¹H NMR (400 MHz, CDCl₃), δ (ppm): 8,91 (1 H, d); 8,85 (1 H, dd); 8,68 (1 H, dd); 8,30 (1 H, d); 8,01 (1 H, dd); 7,96 ( 1H, dd); 7,93 (1 H, dt); 7,49 (1 H, d); 7,45 (1 H, d)

### 26) ethyl 7-(diethylamino)heptanoate

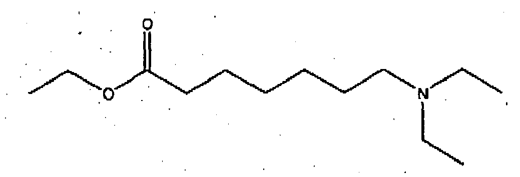

Diethylammine (0.43ml, 4.2 mmol) and sodium iodide(0.14g, 0.9 mmol) were added to a stirred solution of ethyl 7-bromoheptanoate (0.5 g, 2.1 mmol) in toluene (20 ml). The reaction mixture was refluxed for 36 h, cooled to room temperature, and then filtered. The organic layers were washed with saturated Na₂CO₃ solution, dried over MgSO4 and concentrated *in vacuo* giving 0.34 g (70%) of ethyl 7-(diethylamino)heptanoate.
**¹H NMR** (400 MHz, DMSOd₆), δ (ppm): 1.0 (6H, t), 1.25 (3H, t,), 1.2-1.3 (4H, m), 1.5-1.6 (2H, m), 1.6-1.7 (2H, m), 2.3 (2H, t,) 2.4 (2H, m,), 2.55 (4H, q), 4.1 (2H,).
**GC-MS:** 229 (M^{-•}) 214, 200, 156.

### 27) 7-(diethylamino)heptanoic acid

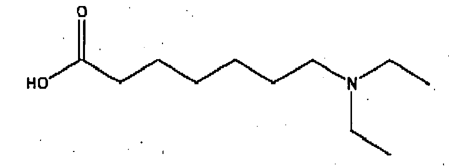

A 1 M aqueous solution of LiOH (4 ml) was added in 10 minutes to a stirred solution of Ethyl 7-bromoheptanoate (0.35 g, 1.5 mmol) in dioxane (15 ml). The reaction mixture was stirred at rt for 2 h, neutralized with diluted HCl and concentrated under reduced pressure until a white solid precipitates. The solid was then suspended to a solution of CHCl₃/1% TEA, filtered and the organic layers was concentrated *in vacuo* giving 0.3 g (quantitative yield) of 7-(diethylamino)heptanoic acid.
**¹H** NMR (400 MHz, DMSOd₆), δ (ppm): 0.9 (t, 3H, CH₃ ammina), 1.19-1.38 (m, 6H, CH₂), 1.45 (qui, 2H, CH₂), 2.1 (t, 2H, CH₂COOH), 2.3 (t, 2H, CH₂), 2.4 (q, 4H; CH₂ ammina).

### 28) 7-(diethylamino)heptanoyl chloride

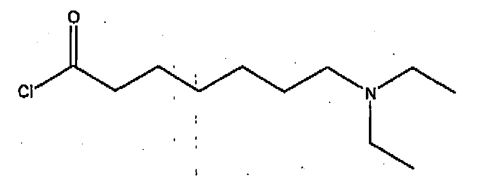

Oxalil chloride (0.13 ml, 1.5 mmol) was added dropwise to a solution of 7-(diethylamino)heptanoicacid (0.3g, 1.5 mmol) in dry THE under inert atmosphere and then two drops of dimethylformammide were added. The reaction mixture was refluxed for 15 minutes, cooled, concentrated *in vacuo* and used without further purification.

### 29) N-(5-(9H-fluoren-7-yl)thiazol-2-yl)-7-(diethylamino)heptanamide (r105)

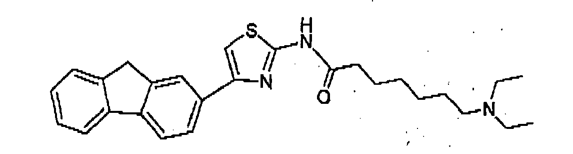

### Procedure 1:

BOP (1 mmol, 0.44 g) and DIEA (3 mmol, 0.5 ml) were added to a stirred solution of 7-(diethylamino)heptanoicacid (1 mmol, 200 mg) in dry DCM (10 ml) under inert atmosphere. After 30 minutes was added the organic amine. The reaction was stirred at rt for additional 18 h and the solvent' was removed under reduced pressure. The crude was purified by flash chromatography (CHCl₃ 97%, NEt₃ 2%, CH₃OH 1 %) giving 220 mg (50%) of N-(5-(9H-fluoren-7-yl)thiazol-2-yl)-7-(diethylamino)heptanamide.

### Procedure 2:

DCC (1.5 mmol, 0.3 g) and HBTU (3 mmol, 1.15 g) were added to a stirred solution of 7-(diethylamino)heptanoicacid (1 mmol, 200 mg) in dry DMF (10 ml) under inert atmosphere. After 5 minutes at rt, a solution of organic amine (1 mmol) in dry DMF (1 ml) was added. The reaction was stirred at RT for 18h, and the solvent was removed under reduced pressure. The crude was purified by flash chromatography (CHCl₃ 97%, NEt₃ 2%, CH₃OH 1%) giving 140 mg (30%) of N-(5-(9H-fluoren-7-yl)thiazol-2-yl)-7-(diethylamino)heptanamide.
**¹H NMR** (400 MHz, CDCl₃), δ (ppm): δ (ppm): 1.03 (t, 6H), 1.15 (m, 4H), 1.36(m, 2H), 1.58(dqu, 2H), 2.22 (t, 2H), 2.34 (m, 2H), 2.53 (q, 4H), 3.95 (s, 2H), 7.19 (s, 1H), 7.32 (t, 1H), 7.39 (t, 1H), 7.56 (d, 1H), 7.83 (m, 3H), 8.01 (s, 1H).

### 30) 3-(methoxycarbonyl)propanoic acid

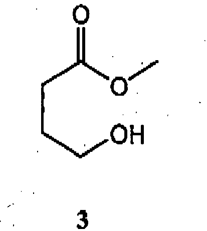

BF3:Et₂O (0.01 mmol) was added dropwise to a solution of dihydrofuran-2,5-dione (2 g, 0.02 mol) in MeOH (12 ml) under inert atmosphere. After 5 minutes at rt the mixture becomes clear, a saturated solution of NaHCO₃ was added, and the aqueous layers was washed with ether. The aqueous solutions was acidified with diluite HCl and extracted with ether. The ethereal phase was washed with brine, dried over MgSO₄, and solvent was removed *in vacuo* giving 3-(methoxycarbonyl)propanoic acid 0.8 g (30%) as a white solid.
1H NMR (400 MHz, CDCl3), δ (ppm):2.59-2.64 (m, 4H), 3.71(s, 3H).

### 31) 3-(5-(9H-fluoren-7-yl)thiazol-2-ylcarbamoyl)propanoate

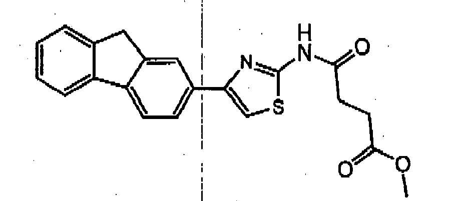

BOP (1.01 g, 2.3mmol) and DIEA (1.2 ml, 6.8 mmol) were added to a stirred solution of 3-(methoxycarbonyl)propanoic acid (0.3 g, 2.3 mmol) in dry DCM (12 mL) under inert atmosphere. After 30 minutes 5-(9H-fluoren-7-yl)thiazol-2-amine (0.6g, 2.3mmol) was added. The reaction was stirred at rt for 18h and the solvent was removed under reduced pressure. The crude was purified by flash chromatography (CHCl₃/CH₃OH 99:1) and crystallized in diethyl ether, giving 440 mg of 3-(5-(9H-fluoren-7-yl)thiazol-2-ylcarbamoyl)propanoate, 50% yield.) as a yellow solid.
1H NMR (400 MHz, CDCl3), δ (ppm):2.60-2.77 (m, 4H), 3.59 (s, 3H), 3.96 (s, 2H), 7.30 (t, 1H), 7.37 (t, 1H), 7.58 (d, 1H), 7.63 (s, 1H), 7.89 (d, 1H), 7.93 (s, 2H), 8.09(s, 1H).

### 32) N-(5-(9H-fluoren-7-yl)thiazol-2-yl)-4-hydroxybutanamide (r108)

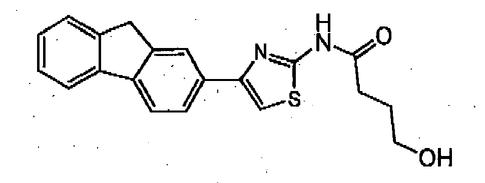

LiCl (0.022 g, 0.52 mmol) was added to a stirred solution of 3-(5-(9H-fluoren-7-yl)thiazol-2-ylcarbamoyl)propanoate (0.1 g, 26 mmol) in 2:1 THF/EtOH (5 mL) at rt under inert atmosphere. After complete dissolution of the inorganic salt NaBH₄ (0.02 g, 0.52 mmol) was added portionwise. After 12 h the reaction mixture was quenched with diluted HCl and the solvent removed under pressure. The crude was purified by flash chromatography (CH₃Cl/CH₃OH 95:5) giving 30 mg (30%) of N-(5-(9H-fluoren-7-yl)thiazol-2-yl)-4-hydroxybutanamide.
¹H NMR (400 MHz, CDCl₃), δ (ppm):1.73 (m, 2H), 2.48(m, 2H), 3.41(m, 2H), 3.96 (s, 2H), 7.30(t, 1H), 7.37 (t, 1H), 7.58 (d, 1H), 7.62 (s, 1H), 7.89(d, 1H), 7.92 (s, 2H), 8.09 (s, 1H).

### 33) N-[5-(9H-Fluoren-2-yl)-thiazol-2-yl]-3-(4-methyl-piperazin-1-yl)-propionamide

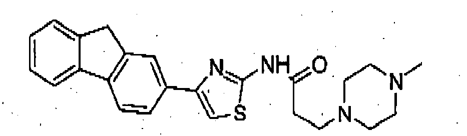

Acryloyl chloride (0.026 ml, 0.32 mmol) was added dropwise to a stirred solution of 5-(9H-fluoren-7-yl)thiazol-2-amine (40 mg, 0.15 mmol) and Et₃N (0.046 ml, 0.32 mmol) in dry DCM (5 ml) under inert atmosphere. After 20 minutes, N-methyl piperazine (0.0134 ml, 0.12 mmol) was added. The reaction was stirred at rt for 12, the solvent was removed reduce pressure, dissolved in EtOAc and washed with aqueous Na₂CO₃. The organic layers were dried with MgSO₄ and concentrated *in vacuo*. The crude was purified by flash chromatography (CH₂Cl₂/EdtOH/Et₃N 91:8:1), giving N-[5-(9H-Fluoren-2-yl)-thiazol-2-yl]-3-(4-methyl-piperazin-1-yl)-propionamide as a brown solid.
¹H-NMR (CDCl₃, 400 MHz), δ (ppm): 8.10 (1H, bs), 7.92 (2H, m), 7.90 (1H, d), 7.60 (1H, s) 7.40 (2H, m) 3.9 (2H, s), 2.90 (8H, m), 2.83 (2H, t), 2.60 (2H, CH₂), 2.20 (3H, s).

### 34) (4-Methyl-piperazin-1-yl)-acetic acid ethyl ester

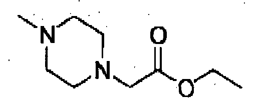

K₂CO₃ (2.48 g, 18 mmol) and N-Methyl piperazine (2 mL, 18 mmol) were added to a solution of Bromo Ethyl Ester (2 mL, 18 mmol) in dry DMF (18 ml) under inert atmosphere. After 30 minutes at 50°C the mixture was diluted with DCM, filtered and the organic layers distilled *in vacuo* giving (4-Methyl-piperazin-1-yl)-acetic acid ethyl ester as a brown oil.
¹H-NMR (CDCl₃, 400 MHz), δ (ppm): 4.18 (2H, q), 3.20 (2H, s), 2.60 (8H, m), 2.26 (3H, s), 1.26 (3H, t)

### 35) 2-(4-methylpiperazin-1-yl)acetic acid

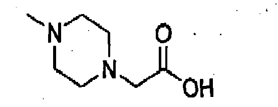

NaOH 3 N (4.8 mL, 14,4 mmol) was added to a stirred solution of (4-Methyl-piperazin-1-yl)-acetic acid ethyl ester (2.26g, 12.13 mmol) in dioxane/water (60:40, 20 mL). After 4 h, the reaction was quenched with diluited HCl, diluted with DCM, filtered and the organic layers distilled *in vacuo* giving (4-Methyl-piperazin-1-yl)-acetic acid as a brown oil.
¹H-NMR (DMSO, 400 MHz), δ (ppm): 4.0 (1H, bs), 3.02 (2H, s), 2.50 (8H, m), 2.16 (3H, s).

### 36) 2-(4-methylpiperazin-1-yl)acetyl chloride

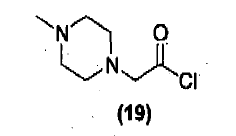

Oxalil chloride (0.3 ml, 3.47 mmol) was added dropwise to a stirred solution of (4-Methyl-piperazin-1-yl)-acetic acid (500 mg, 3.16 mmol) 15 ml of dry THF and placed under inert atmosphere. Two drops of dimethylformammide were added. The reaction mixture was refluxed for 1 h, and the solvent removed under reduced pressure giving 440 mg (80%) (4-Methyl-piperazin-1-yl)-acetyl chloride as a yellow solid.
¹H-NMR (DMSO, 400 MHz), δ (ppm): 3.20 (2H, s), 2.78 (8H, m), 2.60 (3H, s).

### 37) N-(5-(9H-fluoren-7-yl)thiazol-2-yl)-2-(4-methylpiperazin-1-yl)acetamide (r106)

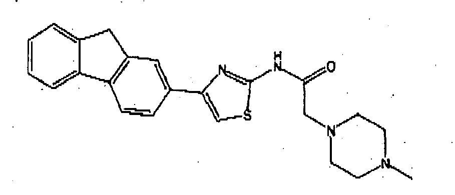

DCC (0.31 g, 1.5 mmol) and HBTU (1.37 g, 3 mmol) were added to a stirred solution of (4-Methyl-piperazin-1-yl)-acetic acid (0.165 g, 1 mmol) in dry DMF (10 ml) under inert atmosphere was placed., and the mixture was stirred at RT for 5 minutes. The solution of 5-(9H-fluoren-7-yl)thiazol-2-amine (0.264 g, 1 mmol) in dry DMF (10 ml) was added dropwise. After 18h at rt, the solvent was removed under reduced pressure. The crude was purified by flash chromatography (CHCl₃/Et₃N/MeOH 95:4:1) and then crystallized from ethyl ether giving 160 mg (40%) of N-(5-(9H-fluoren-7-yl)thiazol-2-yl)-2-(4-methylpiperazin-1-yl)acetamide.
**¹H NMR** (400 MHz, DMSOd₆), δ (ppm): 2.13 (3H, s), 2.32(4H, bs), 2.5 (4H, bs), 3,28 (2H, s), 3.90 (2H, s), 7.31 (1H, t), 7.38 (t, 1H), 7.58 (d, 1H), 7.66 (s, 1H), 7.89 (d, 1H), 7.92 (s, 2H), 8.1 (s, 1H).

### 38) 5-bromofuran-2-carbonyl chloride

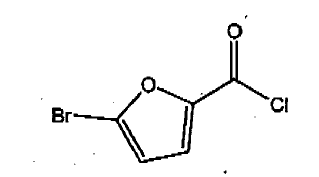

Oxalil chloride (0. 26ml, 2.86mmoli) was added dropwise, to a stirred solution of 5-bromofuran-2-carboxylic acid (500 mg, 2.6 mmol) in dry THF(5 mL) under inert atmosphere. Two drops of dimethylformammide were added. The reaction mixture was refluxed for 15 minutes and the solvent removed under reduced pressure giving 0.48 g (90%) of 5-bromofuran-2-carbonyl chloride as a brown solid.
**¹H NMR** (400 MHz, DMSOd₆), δ (ppm): 7,234 (1 H, s); 6,795 (1 H, s)

### 39) N-(5-(9H-fluoren-2-yl)thiazol-2-yl)-5-bromofuran-2-carboxamide (r88)

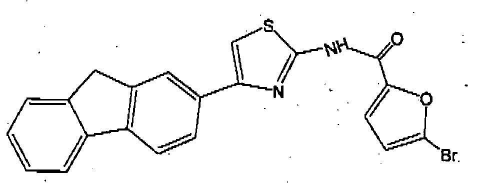

DMF was added to a stirred suspension of 5-bromofuran-2-carboxylic chloride (0.280g, 1.35mmol) and 5-(9H-fluoren-7-yl)thiazol-2-amine (0.3 g, 1.14 mmol) in THF (10 ml), until complete dissolution of the reagents. Et₃N (1 ml, 5.4 mmol) was then added. After 14 h at rt the solvent was removed under reduced pressure, the residue diluted with CHCl₃ and washed with brine. The organic layers were dried over MgSO₄ and concentrated under reduced pressure. The crude was purified by flash chromatography (CH₂Cl₂/EtOH/Et₃N 93:6:1) giving 0.15 g (25%) of N-(5-(9H-fluoren-2-yl)thiazol-2-yl)-5-bromofuran-2-carboxamide.
¹H NMR (400 MHz, DMSOd6), δ (ppm):3.98 (2H, s), 6.87 (1H, d), 7.31(1H, t), 7.38(1H, t), 7.58 (1H, d), 7.64 (1H, d), 7.69 (2H, s), 7.91(1H, d), 7.92-7.99(2H, m), 8.14 (1H, s).

### 40) 4-(4-methylpiperazin -1-carbonyl)- phenyl-boronic acid

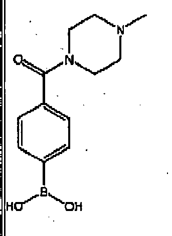

N-methyl-piperazine (0.22 ml, 1.98 mmol) and PyBrOP (0.93 g, 2 mmol) were added to a stirred solution of 4-carboxy phenyl boronic acid (0.33 g, 1.98 mmol) and DIEA (0.7 ml, 4 mmol) in dry DMF (7 ml) under inert atmosphere. After 14 h at rt the solvent was removed under reduced pressure and crude purified by flash chromatography (CH2Cl2/EtOH/Et3N 75:20:5) giving 0.19 g (40%) of 4-(4-methylpiperazin -1-carbonyl)- phenyl-boronic acid.
1H NMR (400 MHz, CD3OD), δ (ppm): 7,85 (2 H, d); 7,66 (2 H, bd)

### 41) 5-[4-(4-Methyl-plperazine-1-carbonyl)-phenyl]-furan-2-carboxylic acid [5-(9H-fluoren-2-yl)-thiazol-2-yl]-amide (r104)

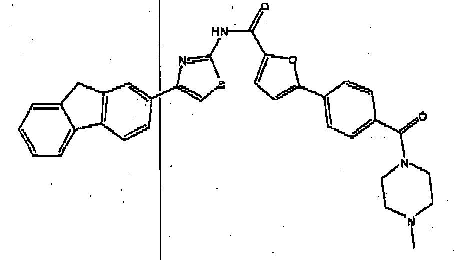

4-(4-methylpiperazin -1-carbonyl)- phenyl-boronic acid (0.170 g, 0.68 mmol) was added to a stirred solution of N-(5-(9H-fluoren-2-yl)thiazol-2-yl)-5-bromofuran-2-carboxamide (0.300 g, 0.68 mmol) in dioxane (50 mL) under inert atmosphere. A solution of K₂CO₃ (0.3 g, 2.04 mmol) in H₂O (10 ml) was then added and the mixture degassed. After addition of a catalytic amount of Pd(PPh₃)₂ (20 mg), the reaction was heated to 100°C for 2 h, cooled to rt and stirred for additional 10 h. Solvents were removed under reduce pressure and the crude purified by flash chromatography (CH₂Cl₂/ MeOH/EtN₃ 79:20:1), giving 50 mg (13%) of 5-[4-(4-Methyl-piperazine-1-carbonyl)-phenyl]-furan-2-carboxylic acid [5-(9H-fluoren-2-yl)-thiazol-2-yl]-amide.
¹H NMR (400 MHz, DMSOd₆), δ (ppm):2.20 (s, 3H), 2.26-2.45 (m, 8H), 3.99, (s, 2H), 7.27-7.34 (m, 2H), 7.39 (t, 1H), 7.51 (d, 2H), 7.60 (d, 1H), 7.66 (d, 1H), 7.74 (d, 1H), 7.87-8.05 (m, 4H), 8,12 (d, 2H), 8.19 (s, 1H).

### 42) N-(5-(9H-fluoren-7-yl)thiazol-2-yl)nicotinamide (r86)

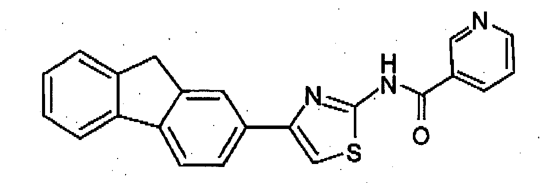

Et₃N (2 ml, 15 mmol) was added to a stirred suspension of nicotinoyl chloride hydrochloride (0.270 g, 1.5 mmol) and 5-(9H-fluoren-7-yl)thiazol-2-amine (0.270 g, 1 mmol) in DCM (15 ml). After 2 d at rt, the solvents were removed under reduced pressure, diluted with DCM and washed with brine. The organic layers were dried with MgSO4 and concentrated *in vacuo*. The crude was purified by flash chromatography (CH₂Cl₂/MeOH/EtN₃, 97:2:1) giving 36 mg (10%) of N-(5-(9H-fluoren-7-yl)thiazol-2-yl)nicotinamide.
1H NMR (400 MHz, CDCl3), δ (ppm): 8,91 (1H, d); 8,69 (1H, s); 8,62 (1H, bs); 8,00 (1 H, bs); 7.89- 7,83 (3 H, m);' 7,60 (2 H, m); 7,42 (2 H, m); 7.24 (1 H, s) 4,01 (2 H, s).

### 43) N-(5-(9H-fluoren-7-yl)thiazol-2-yl)-2-iodobenzamide (r87)

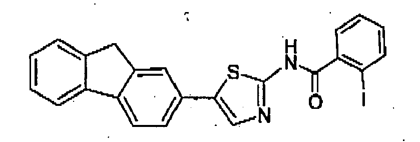

Until complete dissolution of the reagents DMF, was added to a stirred suspension of 2-iodobenzoyl chloride (0.390 g, 1.5 mmol) and 5-(9H-fluoren-7-yl)thiazol-2-amine (0.3 g, 1.14 mmol) in dry THF (10 ml) under inert atmosphere. Et₃N (1.8 ml, 12 mmol) was then added. After 14 h at rt the solvents were removed under reduced pressure, and the residue was diluted with trichloromethane and washed with brine. The organic layers were dried with MgSO4 and concentrated under reduced pressure. The crude was purified by flash chromatography (CH₂Cl₂/MeOH/Et₃N, 97:2:1) giving 110 mg of N-(5-(9H-fluoren-7-yl)thiazol-2-yl)-2-iodobenzamide.
¹H NMR (400 MHz, CDCl₃), δ (ppm): 8.30 (1 H, s), 8.10 (1 H, s), 8.00- 7.90 (2 H, m), 7.82- 7.77 (2 H, m), 7.420-7.29 (3 H, m), 7.24 (1 H, s), 7.17 (1 H, m), 7.025 (1 H, s); 3.93 (2H,s).

### 44) 2-(methylthio)thiazole

A solution of *n*-BuLi (2.5 M, 2.1 ml, 5.2 mmol) in hexanes was added dropwise to a stirred solution of thiazole (0.36 ml, 5 mmol) in dry THF (20 ml) at -78°C under inert atmosphere. After 1 h, 1,2-dimethyldisulfane (5.2 mmol) was added. The reaction mixture was stirred for additional 2 h, quenched with saturated Na₂CO₃, extracted with ether, and the organic layers dried with MgSO₄. The crude was purified by distillation under reduce pressure giving 0.52 g (80%) of 2-(methylthio)thiazole.
¹H NMR (400 MHz, CDCl₃), δ (ppm): 7,65 (1H, d); 7,20 (1H, d); 2,70 (3H, s);

### 45) 5-(trimethylstannyl)-2-(methylthio)thiazole

A solution of *n*-BuLi (2.5 M, 2.1 ml, 5.2 mmol) in hexanes was added dropwise to a stirred solution of 2-(methylthio)thiazole (0.52 g, 4 mmol) in dry THF (20 ml) at -78°C under inert atmosphere. After 1 h Me₃SnCl (5 mmol, 1g) was added portionwise. After 14 h the reaction was quenched with saturated Na2CO3, extracted with ether and the organic layers were dried with MgS04. The crude was purified by distillation under reduce pressure giving 0.90 g (70%) of 5-(trimethylstannyl)-2-(methylthio)thiazole.
¹H NMR (400 MHz, CDCl₃), δ (ppm): 7,57 (1H, s); 2,69 (3H, s); 0,38 (3H, s)

### 46) 1-(9-benzenesulfonyl-9H-carbazol-2-yl)-ethanone 1:

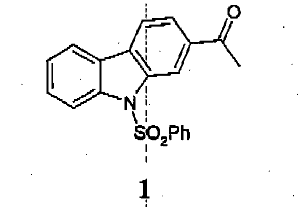

60% NaH in paraffin (21.2 mg, 0.53 mmol) was added to a stirred solution of 2-acetylcarbazole (100 mg, 0.48 mmol) in 3.5 mL of anhydrous THF at 0°C. After stirring at 0°C for 20 min, benzenesulfonyl chloride (74 µL, 0.57 mmol) was added dropwise. The reaction mixture was stirred for 12 hours and then poured with 5% aqueous NaHCO₃ and extracted with EtOAc. The combined organic layers were dried (MgSO₄), and the solvent was removed under reduced pressure. The crude product was purified by recrystallization from EtOAc to furnish the desired compound in 80% (134 mg, 0.383 mmol) as a white solid; MS (ESI) *m*/*z* 350 [M+H⁺]; ¹H NMR (CDCl₃; 300 MHz) δ 8.93 (s, 1H), 8.36 (d, 1H, J= 8.5 Hz), 8.02-7.95 (m, 3H), 7.83 (d, 2H, *J* ≈ 7.9 Hz), 7.57 (ddd, 1H, *J* = 7.8 Hz, *J* = 7.3 Hz, *J* = 1.1 Hz), 7.48-7.32 (m, 4H), 2.75 (s, 3H); ¹³C NMR (CDCl₃; 300 MHz) δ 197.5 (C), 139.5 (C), 138.0 (C), 137.6 (C), 136.0 (C), 134.0 (CH), 130.1 (C), 129.1 (2 CH), 128.8 (CH), 126.4 (2 CH), 125.3 (CH), 124.3 (CH), 124.0 (CH), 120.8 (CH), 119.9 (CH), 115.3 (CH), 115.1 (C), 26.9 (CH₃).

### 47) 4-(9-benzenesulfonyl-9H-carbazol-2-yl)-thiazol-2-ylamine 3:

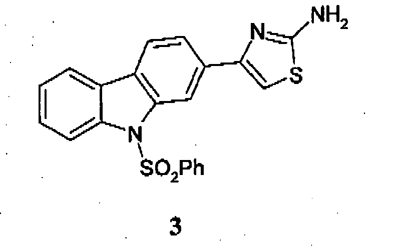

To a suspension of CuBr₂ (1.28 g, 5.72 mmol) in EtOAc (13 mL) was added a solution of 2-acetyl-1-phenylsulfonyl-1*H*-carbazole (1) (1 g, 2.86 mmol) in EtOAc (13 mL) under argon at room temperature. The mixture was stirred under reflux for 90 min. 645 mg of CuBr₂ (2.88 mmol) were added in the mixture to allow complete conversion to the monobrominated derivative. After cooling, precipitates were removed by filtration and washed with ethyl acetate. Combined filtrates were washed with saturated aqueous NaHCO₃ solution and brine, dried over anhydrous MgSO₄ and evaporated to dryness *in vacuo*. The product 2 was used immediately without further purification.

To a stirred suspension of 2 (1.10 g, 2.57 mmol) in EtOH (15 mL) was added thiourea (195 mg, 76.12 mmol) and the mixture was heated at 70°C for 2h. After cooling to room temperature, the solvent was evaporated to dryness. The resulting solid was stirred in a mixture of EtOAc/saturated aqueous NaHCO₃ solution (2/1) until dissolution, and then extracted with EtOAc. The organic layer was washed with brine, dried over anhydrous MgSO₄, filtrated and solvent was removed under reduced pressure. The crude product was purified by flash chromatography (EtOAc) to afford 3 in 69% yield (801 mg, 1.968 mmol) as a yellow solid; MS (ESI) *m*/*z* 408 [M+H⁺]; ¹H NMR ((CD₃)₂CO; 300 MHz) δ 8.92 (d, 1H, *J* = 0.8 Hz), 8.33 (d, 1H, *J* = 8.5 Hz), 8.05 (d, 1H, *J* = 6.0 Hz), 8.03 (d, 1H, *J* = 8.1 Hz), 7.94-7.89 (m, 3 H), 7.62-7.38 (m, 5H), 7.13 (s, 1H), 6.60 (bs, 2H); ¹³C NMR (DMSO-d₆; 300 MHz) δ 168.4 (C), 149.6 (C), 138.2 (C), 137.8 (C), 136.6 (C), 134.8 (C), 134.7 (CH), 129.8 (2xCH), 127.6 (CH), 126.0 (2xCH), 125.8 (C), 124.7 (C), 124.5 (CH), 122.1 (CH), 120.7 (CH), 120.6 (CH), 114.6 (CH), 111.8 (CH), 102.7 (CH).

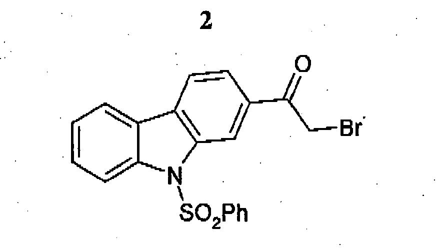

Intermediate (**2**) was isolated as a white solid; MS (EI) *m*/*z* 427, 429 [M^{+·}; ⁷⁹Br, ⁸¹Br]; ¹H NMR (CDCl₃; 300 MHz) δ 8.96 (s, 1H), 8.38 (d, 1H, *J =* 9.0 Hz), 8.02-7.95 (m, 3H), 7.86 (dd, 2H, *J* ≈ 7.4 Hz), 7.57 (ddd, 1H, *J* = 7.8 Hz, *J* = 7.3 Hz, *J* = 1.1 Hz), 7.48-7.32 (m, 4H), 4.59 (s, 2H); ¹³C NMR (DMSOd₆; 300 MHz) δ 191.1(C), 138..7 (C), 137.2 (C), 136.2 (C), 135.0 (CH), 133.0 (C), 130.1 (C), 129.8 (2xCH), 129.5 (CH), 126.3 (2xCH), 125.0 (CH), 124.8 (CH), 124.7 (C), 121.9 (CH), 121.0 (CH), 114.8 (CH), 114.7 (CH), 34.5 (CH₂).

### 48) General Procedure for the preparation of N-acyl substituted thiazole

To a 0.15 M stirred suspension of the (4-aminothiazol-2-yl)-1-phenylsulfonyl-1*H*-carbazole **3** in anhydrous CH₂Cl₂ was added anhydrous pyridine (2 eq) and acyl chloride (1.5 eq) at room temperature under inert atmosphere. The mixture was stirred at room temperature until completion of the reaction (followed by T.L.C.). The resulting mixture was quenched with H₂O and extracted three times with CH₂Cl₂. The resulting organic layers were washed with NH₄Cl saturated aqueous solution and brine, dried over MgSO_{4,} filtered and solvents were removed under reduced pressure.

### 49) N-[4-(9-Benzenesulfonyl-9H-carbazol-2-yl)-thiazol-2-yl]-acetamide 4

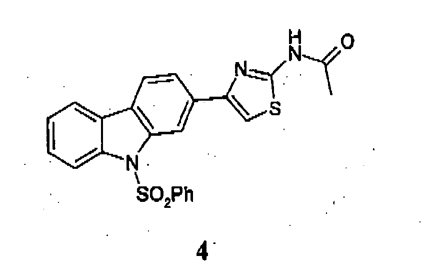

The desired compound is obtained in 64% yield (70 mg from 100 mg of 3, 0.156 mmol) by flash chromatography on silica gel (CH₂Cl₂) as a yellow solid; MS (ESI) *m*/*z* 448 [M+H⁺]; ¹H NMR (CDCl₃; 300 MHz) δ 9.57 (bs, 1H), 8.83 (s, 1H), 8.32 (d, 1H, *J* = 8.5 Hz), 7.93-7.88 (m, 3H), 7.81 (d, 2H, *J* ≈ 7.9 Hz), 7.50 (ddd, 1H, *J* = 8.2 Hz, *J* = 7.9 Hz, *J* = 1.1 Hz),7.44-7.39 (m, 2H), 7.31-7.27 (m, 3H), 2.02 (s, 3H); ¹³C NMR (DMSO-d₆; 300 MHz) δ 168.8 (C); 158.8 (C), 148.4 (C), 138.2 (C), 137.8 (C), 136.5 (C), 134.8 (CH), 134.1 (C), 129.8 (2xCH), 127.8 (CH), 126.0 (2xCH), 125.6 (C), 125.2 (C), 124.5 (CH), 122.3 (CH), 121.0 (CH), 120.7 (CH), 114.6 (CH), 111.7 (CH), 108.9 (CH), 22.5 (CH₃).

### 50) N-[4-(9-Benzenesulfonyl-9H-carbazol-2-yl)-thiazol-2-yl]-benzamide 5

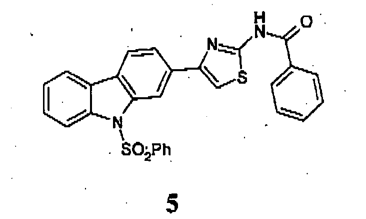

The desired compound is obtained in 61% yield (76 mg from 100 mg of 3, 0.1.49 mmol) by flash chromatography on silica gel (CHCl₃/Pct. Eth. 1/1) as a yellow solid; MS (ESI) *m*/*z* 510 [M+H⁺], 1018 [2M+H⁺]; ¹H NMR (CDCl₃; 300 MHz) δ 9.96 (bs, 1H), 8.91 (s, 1H), 8.32 (d, 1H, *J =* 8.3 Hz), 8.03 (d, 2H, *J* ≈ 7.7 Hz), 7.91-7.84 (m, 5H), 7.63-7.29 (m, 9H); ¹³C NMR (CDCl₃; 300 MHz) δ 164.9 (C), 158.8 (C), 150.0 (C), 139.0 (C), 138.9 (C), 137.9 (C), 133.0 (CH), 133.8 (C), 133.0 (CH), 131.9 (C), 129.2 (2x CH), 129.0 (2xCH), 127.2 (3xCH), 126.6 (2xCH), 126.3 (CH), 126.2 (C), 124.2 (CH), 122.3 (CH), 120.3 (CH), 120.2 (CH), 115.2 (CH), 112.9 (CH), 108.8 (C).

### 51) Biphenyl-4-carboxylic acid [4-(9-Benzenesulfonyl-9H-carbazol-2-yl)-thiazol-2-yl]-amide 6

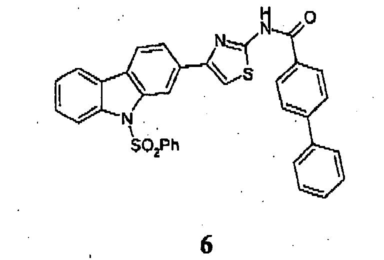

The desired compound is obtained in 60% yield (174 mg from 200 mg of 3, 0.297 mmol) by flash chromatography on silica gel (CHCl₃/toluène 1/1) as a yellow solid; MS (ESI) *m*/*z* 586 [M+H⁺], 1170 [2M+H⁺]; ¹H NMR (CDCl₃; 300 MHz) δ 9.89 (bs, 1H), 8.92 (s, 1H), 8.32 (d, 1H, *J* = 8.3 Hz), 8.08 (d, 1H, *J* = 8.5Hz), 7.93-7.84 (m, 5H), 7.75 (d, 2H, *J* ≈ 8.3 Hz), 7.64 (d, 2H, *J* ≈ 7.0 Hz), 7.52-7.29 (m, 9H); ¹³C NMR (CDCl₃; 300 MHz) δ 164.8 (C), 159.0 (C), 150.0 (C), 154.4 (C), 139.5 (C), 138.8 (C), 138.7 (C), 137.9 (C), 133.9 (CH), 133.8 (C), 130.4 (C), 129.2 (2xCH), 129.0 (2xCH), 128.3 (CH), 128.0 (2xCH), 127.5 (CH), 127.3 (2xCH), 127.2 (2xCH), 126.5 (2xCH), 126.1 (C), 126.0 (C), 124.1 (CH), 122.2 (CH), 120.2 (CH), 120.1 (CH), 115.1 (CH), 112,8 (CH), 108.8 (CH).

### 52) General Procedure for the coupling peptide

To a 2 M stirred mixture of the carboxylic acid in anhydrous DMF was added a 0.5 M solution of (4-aminothiazol-2-yl)-1-phenylsulfonyl-1*H-*carbazole 3 in anhydrous DMF, a 2 M solution of EDCI in dry DMF and 0.4 eq of DMAP, at room temperature under inert atmosphere. The reaction mixture was stirred at room temperature for 12 hours and DMF was evaporated *in vacuo.*

### 53) N-[4-(9-Benzenesulfonyl-9H-carbazol-2-yl)-thiazol-2-yl]-4-(4-methyl-piperazin-1-ylmethyl)-benzamide 7

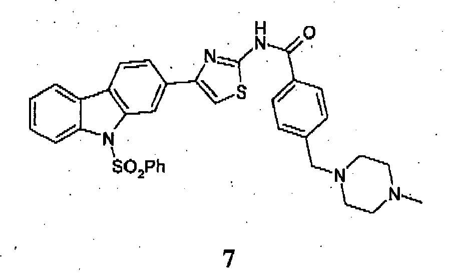

The desired compound is obtained in 24% yield (56 mg from 150 mg of 3, 0.090 mmol) by flash chromatography on silica gel (CH₂Cl₂/MeOH 95/5) as a white solid; MS (ESI) *m*/*z* 622 [M+H⁺]; ¹H NMR (CDCl₃; 300 MHz) 9.86 (bs, 1H) 8.91 (s, 1H), 8.32 (d, 1H, *J* = 8.3 Hz), 7.96-7.82 (m, 7H), 7.51-7.31 (m, 8H), 3.58 (s, 2H), 2.51 (br. m, 8H), 2.32 (s, 3H); ¹³C NMR (DMSO-d₆; 300 MHz) δ 164.9 (C), 1.58.5 (C), 1.48.7 (C), 142.8 (C), 138.1 (C), 137.8 (C), 136.6 (C), 134.3 (CH), 133.9 (C), 130.6 (C), 129.4 (2xCH), 128.4 (2xCH), 127.9 (2xCH), 127.4 (CH), 125.7 (2xCH), 125.4 (C), 125.0 (C), 124.2 (CH), 122.2 (CH), 120.5 (CH), 120.3 (CH), 114.3 (CH), 111.6 (CH), 109.2 (CH), 60.8 (CH₂), 53.7 (2xCH₂), 51.2 (2xCH₂), 44.3 (CH₃).

### 54) 5-Bromo-furan-2-carboxylic acid [4-(9-benzenesulfonyl-9H-carbazol-2-yl)-thiazol-2-yl]-amide 8;

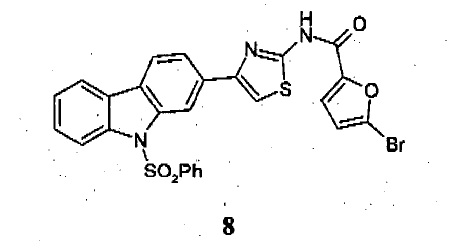

The desired compound is obtained in 20% yield (189 mg from 653 mg of 3, 0.328 mmol) by flash chromatography on silica gel (CH₂Cl₂) as a yellow solid; MS (ESI) *m*/*z* 577, 579 [M+H⁺; ⁷⁹Br, ⁸¹Br]; ¹H NMR (CDCl₃; 300 MHz) δ 10.13 (bs, 1H), 8.89 (s, 1H) 8.31 (d, 1H, *J* = 8.3 Hz), 7.90-7.80 (m, 5H), 7.48 (ddd, 1H, *J* = 8.5 Hz, *J* = 7.4 Hz, *J* = 1.3 Hz), 7.44 (ddd, 1H, *J* = 7.5 Hz, *J* = 1.2 Hz, *J* = 1.1 Hz), 7.41-7.24 (m, 5H), 6.49 (d, 1H, *J* = 3.6 Hz); ¹³C NMR (DMSO-d₆; 300 MHz) δ 158.1 (C), 155.0 (C), 149.0 (C), 147.5 (C), 138.3 (C), 137.9 (C), 136.5 (C), 134.8 (CH), 134.0 (C), 129.8 (2xCH), 127.9 (CH), 127.5 (C), 126.1 (2xCH), 125.7 (C), 125.4 (C), 124.6 (CH), 122.5 (CH), 121.0 (CH), 120.8 (CH), 118.9 (CH), 114.7 (CH), 114.5 (CH), 111.8 (CH), 109.8 (CH).

### 55) 5-[4-(Piperazine-1-carbonyl)-phenyl]-furan-2-carboxylic acid [4-(9-benzenesulfonyl-9H-carbazol-2-yl)-thiazol-2-yl]-amide 9

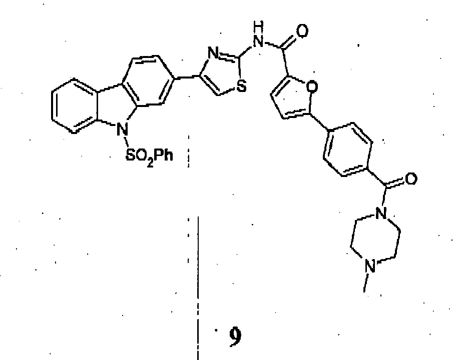

The compound **(8)** (113 mg, 0.19 mmol), [1-(4-methyl)-piperazinyl carbonyl] boronic acid (56 mg, 0.22 mmol), Pd(PPh₃)₄ (34 mg, 0.03 mmol) and K₂CO₃ (79 mg, 0.57 mmol) in 1,4 dioxane (8 mL) and H₂O (2mL) was degased with argon and then stirred at 100°C for 15 hours. Solvents were removed under reduced pressure. The crude product was purified by flash chromatography on silica gel (CH₂Cl₂/MeOH 95/5) to afford 3 in 64% yield (90 mg, 0.128 mmol) as green foam; MS (ESI) *m*/*z* 702 [M+H⁺]; ¹H NMR (CDCl₃; 300 MHz) δ 10.36 (bs, 1H), 8.84 (d,1H, *J* = 0.8 Hz), 8.29 (d, 1H, *J* = 8.3 Hz), 7.86 (d, 2H, *J* ≈ 7.9 Hz), 7.82-7.78 (m, 4H), 7.76 (s, 1H), 7.52-7.27 (m, 9H), 6.80 (d, 1H, *J* = 3.8 Hz), 3.82 (br. m, 2H), 3.49 (br. m, 2H), 2.50 (br. m, 2H), 2.39 (br. m, 2H), 2.33 (s, 3H); ¹³C NMR ((CD₃)₂CO; 300 MHz) δ 169.6 (C), 158.7 (C), 157.0 (C), 156.5 (C), 150.5 (C), 146.6 (C), 139.7 (C), 139.4 (C), 138.2 (C), 137.5 (C), 135.2 (CH), 132.8 (CH), 132.7 (CH), 132.7 (CH), 132.5 (CH), 131.1 (C), 130.3 (2xCH), 129.5 (CH), 129.3 (CH), 128.7 (2xCH), 128.4 (CH), 127.2 (2xCH), 127.1 (C), 126.7 (C), 125.5 (2xCH), 125.3 (CH), 123.2 (CH), 121.3 (CH), 121.2 (CH), 119.8 (CH), 115.8 (CH), 113.3 (CH), 109.8 (CH), 109.7 (CH), 46.1 (CH₃).

### 56) General procedure for the deprotection of N-sulfonyl with tetrabutylammonium fluoride

To a 1 M mixture of compound **(3-9)** in anhydrous THF was added 4 eq of TBAF (1.0 M solution in THF), under inert atmosphere. The mixture was refluxed until completion of the reaction (followed by T.L.C, 2-3 hours). Solvent was removed and the residue was dissolved in CH₂Cl₂. The organic layer was washed with water, brine, dried over anhydrous MgSO₄, filtered and the solvent was removed under reduced pressure.

### 57) 4-(9H-Carbazol-2-yl)-thiazol-2-ylamine 10 (r156)

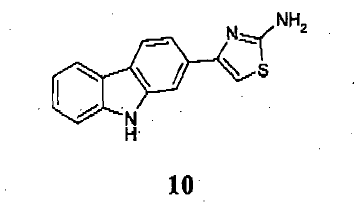

The residue was purified by flash chromatography on silica gel (CHCl₃/MeOH 95/5) and recrystallized from EtOH to furnish the desired compound as a white solid in 29% yield (8.4 mg, 0.032 mmol); MS (ESI) *m*/*z* 266 [M+H⁺]; ¹H NMR ((CD₃)₂CO; 300 MHz) δ 11.11 (bs, 1H), 8.84 (m, 3H), 8.47 (dd, 1H, *J* = 8.1 Hz, J= 1.7 Hz), 8.25 (d, 1H, *J* = 8.1 Hz), 8.12 (ddd, 1H, *J* = 7.8 Hz, *J* = 7.1 Hz, *J* = 0.6 Hz), 7.95 (ddd, 1H, *J* = 7.8Hz *J* = 7.1 Hz, *J* = 0.6 Hz), 7.73 (s, 1H), 7.18 (bs, 2H).

### 58) N-[4-(9H-Carbazol-2-yl)-thiazol-2-yl]-acetamide 11

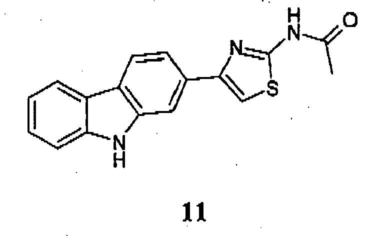

The residue was recrystallized from EtOH to furnish the desired compound as a white solid in 33% yield (15 mg, 0.049 mmol); MS (ESI) *m*/*z* 308 [M+H⁺]; ¹H NMR ((CD₃)₂CO); 300 MHz) δ 11.04 (bs, 1H), 10.37 (bs, 1H), 8.15-8.10 (m, 3H), 7.76 (dd, 1H, *J* = 1.5 Hz, *J* = 8.3 Hz), 7.52 (d, 1H, *J* = 8.1 Hz), 7.49 (s, 1H), 7.37 (ddd, 1H, *J* = 7.7 Hz, *J* = 7.0 Hz, *J* = 0.9 Hz), 7.17 (ddd, 1H, *J* = 7.7 Hz, *J* = 7.0 Hz, *J* = 0.9 Hz), 2.29 (s 3H); HRMS calcd for C₁₇H₁₃N₃OS [M+H]⁺ 308.0858 found 308.0857

### 59) N-[4-(9H-Carbazol-2-yl)-thiazol-2-yl]-benzamide 12 (r158)

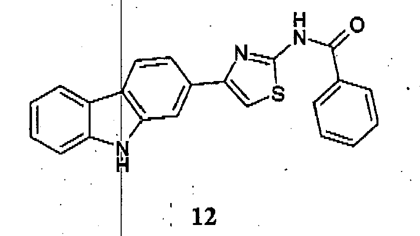

The residue was recrystallized from EtOH to furnish the desired compound as a white solid in 42% yield (17 mg, 0.046 mmol); MS (ESI) *m*/*z* 370 [M+H⁺]; ¹H NMR ((CD₃)₂CO); 300 MHz) δ 11 48 (bs, 1H), 10.44 (bs, 1H), 8.23 (d, 2H, *J* ≈ 7.0 Hz), 8.16-8.11 (m, 3H), 7.81 (dd, 1H, *J* = 8.2 Hz, *J* = 1.5 Hz), 7.62 (m, 4H), 7.51 (d, 1H, *J* = 8.2 Hz), 7.39 (ddd, 1H, *J* = 7.6 Hz, *J* = 7.3 Hz, *J* = 1.0 Hz), 7.18 (ddd, 1H, *J* = 7.6 Hz, *J* = 7.3 Hz, *J* = 1.0 Hz); HRMS calcd for C₂₂H₁₅N₃OS [M+H]⁺ 370.1014 found 370.1013.

### 60) Biphenyl-4-carboxylic acid 4-(9H-carbazol-2-yl)-thiazol-2-yl]-amide 13 (r169)

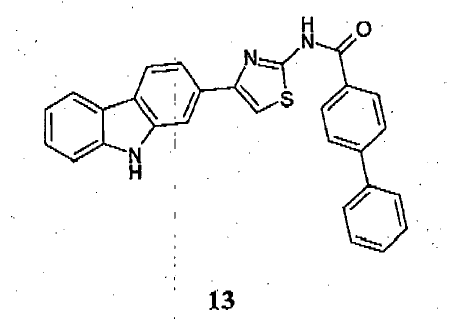

The residue was obtained by hot filtration from CHCl₃ to furnish the desired compound as a white solid in 10% yield (14 mg, 0.031 mmol); MS (ESI) *m*/*z* 446 [M+H⁺]; ¹H NMR (DMSO-d₆; 300 MHz) δ 12.87 (bs, 1H), 11.37 (bs, 1H), 8.26 (d, 2H, *J* ≈ 8.4 Hz), 8.17-8.09 (m, 3H), 7.88 (d, 2H, *J* ≈ 8.4 Hz ), 7.81-7.75 (m, 4H), 7.55-7.36 (m, 5H), 7.17(t, 1H, *J* = 7.3 Hz); HRMS calcd for C₂₈H₁₉N₃OS [M+H]⁺ 446.1327 found 446.1326.

### 61) N-[4-(9H-carbazol-2-yl)-thiazol-2-yl]-4-(4-methyl-piperazin-1-ylmethyl)-benzamide 14

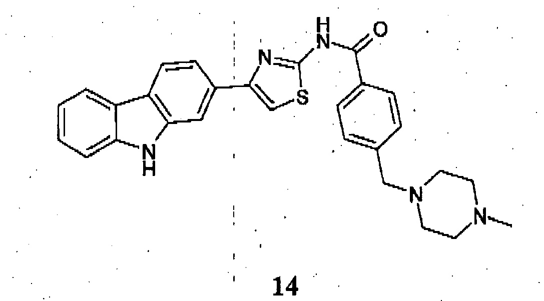

The residue was recrystallized from EtOH to furnish the desired compound as a white solid in 50% yield (20 mg, 0.041 mmol); MS (ESI) *m*/*z* 482 [M+H⁺]; ¹H NMR (DMSO-d₆; 300 MHz) δ 12.73 (bs, 1H), 11.35 (bs, 1H), 8.16-8.08 (m, 5H), 7.77 (dd, 1H, *J* = 8.1 Hz, *J* = 1.3 Hz), 7.71 (s, 1H), 7.51-7.46 (m, 3H), 7.38 (td, 1H, *J* = 0.9 Hz, *J* = 7.3 Hz), 7.16 (td, 1H, *J* = 0.9 Hz, *J* = 7.3 Hz), 3.55 (s, 2H), 2.39 (br. m, 4H), 2.34 (br. m, 4H), 2.15 (s, 3H); HRMS calcd for C₂₈H₂₇N₅OS [M+H]⁺ 482.2015 found 482.2015.

### 62) 5-Bromo-furan-2-carboxylic acid [4-(9H-carbazol-2-yl)-thiazol-2-yl]-amide 15 (r170)

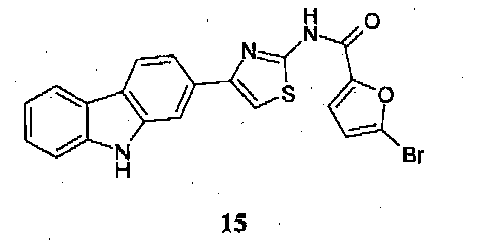

The residue was recrystallized from EtOH to furnish the desired compound as a yellow solid in 66% yield (25 mg, 0.057 mmol); MS (ESI) *m*/*z* 438, 440 [M+H⁺; ⁷⁹Br, ⁸¹Br]; ¹H NMR (DMSO-d₆; 300 MHz) δ 12.83 (bs, 1H), 11.34 (bs, 1H), 8.16-8.10 (m; 2H), 8.05 (d, 1H, *J* = 1.0 Hz), 7.77-7.73 (m, 3H), 7.50 (d, 1H, *J* = 8.1 Hz), 7.38 (ddd, 1H, *J* = 7.6 Hz, *J* = 7.2 Hz, *J* = 1.0 Hz), 7.16 (ddd, 1H, *J* = 7.6 Hz, *J* = 7.2 Hz, *J* = 1.0 Hz), 6.91 (d, 1H, *J* = 3.6 Hz).

### 63) 5-[4-(Piperazine-1-carbonyl)-phenyl]-furan-2-carboxylic acid [4-(9H-carbazol-2-yl)-thiazol-2-yl]-amide 16

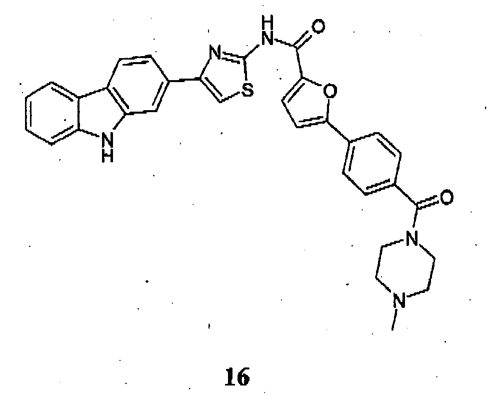

The residue was filtrated after trituration in MeOH to furnish the desired compound as a white solid in 39% yield (22 mg, 0.039 mmol); MS (ESI) *m*/*z* 562 [M+H⁺]; ¹H NMR (DMSO-d₆ at 70°C; 300 MHz) δ 13.06, (bs, 1H), 11.14 (bs, 1H), 8.15-8.08 (m, 5H), 7.77 (dd, 1H, *J* = 8.1 Hz, *J* = 1.5 Hz), 7.65-7.63 (m, 2H), 7.54-7.49 (m, 3H), 7.39 (ddd, 1H, *J* = 7.5 Hz, *J* = 5.7 Hz, *J* = 1.1 Hz), 7.25 (d, 1H, *J* = 3.6 Hz), 7.17 (ddd, 1H, *J* = 7.54 Hz, *J* = 5.7 Hz, *J* = 1.1 Hz), 3.5 (br. m, 4H), 2.36 (br. m, 4H), 2.23 (s, 3H); HRMS calcd for C₃₂H₂₇N₅O₃S [M+H]⁺ 562.1913 found 562.1911.

| No. | Compound | Formula | Synthesis |
|---|---|---|---|
| **64)** | **r113** | 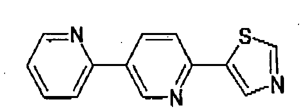 | General synthesis strategies (page 8) starting from (11) |
| **65)** | **r121** | 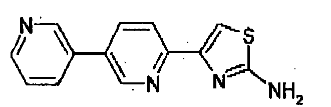 | General synthesis strategies |
| **66)** | **r122** | 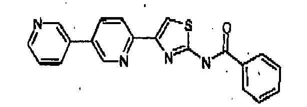 | General synthesis strategies |
| **67)** | **r124** | 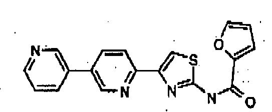 | General synthesis strategies |
| **68)** | **r201** | 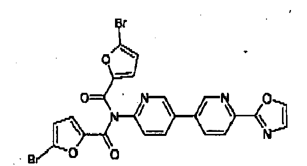 | General synthesis strategies as described for r200 (17) using 2 equivalent excess of 5-bromofuran-2-carbonyl chloride (38) |
| **69)** | **R89** | 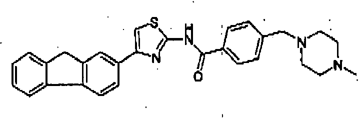 | Synthesis similar to r104 (41) with a final step consisting in a keton reduction of carbonyl piperazine moiety. |
| **70)** | **R233** | 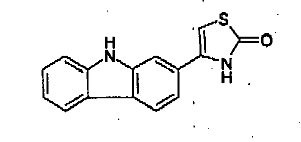 | General synthesis for 4-(9H-Carbazol-2-yl)-thiazol (NH2 of r156 exchanged with -OH) |

### 2. Alk kinase inhibitory activity

### Method: ELISA-based in vitro kinase assay

Recombinant ALK kinase was expressed in Sf9 insect cells using the pBlueBacHis2C baculovirus vector system and purified using an anion exchange Fast Flow Q-sepharose column (Amersham-Pharmacia Biotech) followed by HiTrap™-nickel affinity column (Amersham-Pharmacia Biotech). Purified ALK protein was used to screen inhibitors in the ELISA-based kinase assay. A Nunc Immuno 96 well plate was incubated overnight at 37°C with coating solution (125 µl/ well) containing ALK peptide substrate (ARDIYRASFFRKGGCAMLPVK) at various concentrations in PBS. Wells were then washed with 200 µl of wash buffer (PBS-Tween 0.05%) and left to dry for at least 2 hours at 37°C. The kinase reaction was performed in the presence of 50 mM Tris pH 7.5, 5 mM MnCl₂, 5 mM MgCl₂, 0.3 mM ATP and purified rALK in a total volume of 100 µl/well at 30°C for 15 minutes. For inhibitor testing the reaction mix was preincubated with the inhibitor or solvent control for 10 mins at room temperature before transferring to the ELISA plate. After the reaction wells were washed 5 times with 200 µl of wash buffer. Phosphorylated peptide was detected using 100 µl/well of a mouse monoclonal anti-phosphotyrosine antibody (clone 4G10 UpstateBiotech Ltd) diluted 1:2000 in PBS + 4% BSA. After 30 minutes incubation at room temperature the antibody was removed and wells were washed as described above. 100 µl of a secondary antibody (anti-mouse IgG, Horseradish Peroxidase linked whole antibody, Amersham Pharmacia Biotech) diluted 1:1000 in PBS + 4% BSA was added to each well and the plate was incubated again for 30 minutes at room temperature before washing as above. The plate was developed using 100 µl/well TMB Substrate Solution (Endogen) and the reaction was stopped by adding an equal volume of H₂SO₄ 0.36 M. Finally, the absorbance was read at 450 nm using an Ultrospec® 300 spectrophotometer (Amersham-Pharmacia Biotech). The concentration of the test solution showing 50% inhibition as compared with the control was expressed as IC₅₀.

### Results from ELISA kinase assay

**Table 1: IC₅₀ values on ALK**

| compound | Identifier | IC₅₀ (µM) | Formula |
|---|---|---|---|
| **r19** | MFCD00045579 | 1.2 | 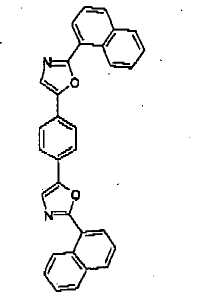 |
| **r35** | MFCD01765083 | 7.7 ± 0.2 | 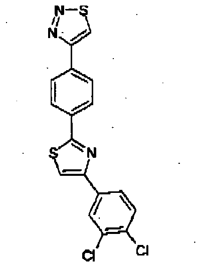 |
| **r36** | MFCD01765086 | 27 | 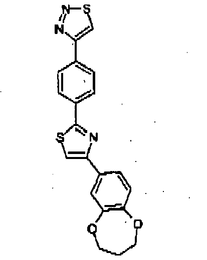 |
| **r37** | MFCD01765092 | 1.5 ± 0.3 | 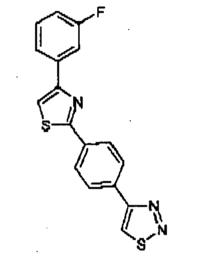 |
| **r68** | MFCD01765084 | 3.1 ± 0.34 | 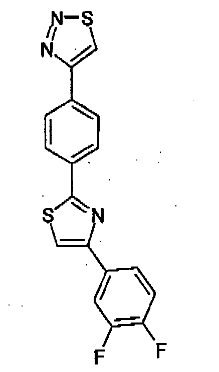 |
| **r69** | MFCD01765087 | 9.3 ± 2.9 | 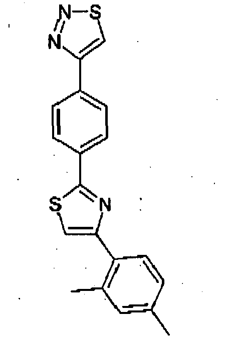 |
| **r70** | MFCD01765088 | 5.5 ± 2.3 | 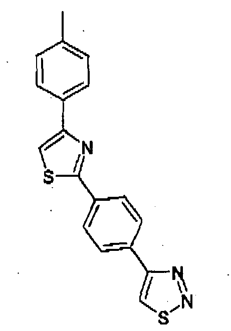 |
| **r75** | MFCD01934429 | 8.8 ± 0.85 | 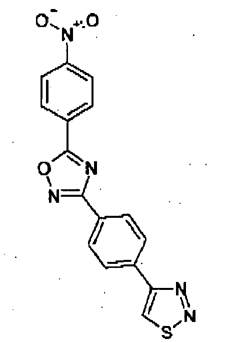 |
| **r78** | MFCD01934431 | 103 | 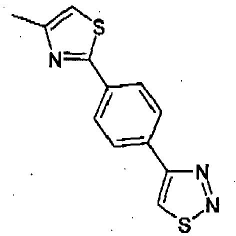 |
| **r79** | MFCD00113424 | 24 ± 1.5 | 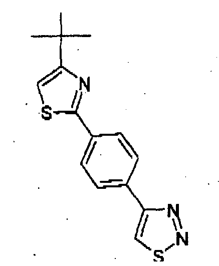 |
| **r80** | MFCD00113296 | 48 | 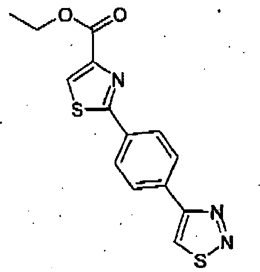 |
| **r81** | MFCD00205741 | 46.5 | 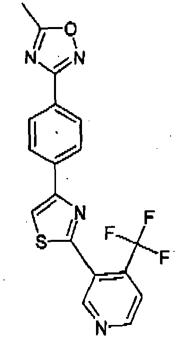 |
| **r43** | MFCD01764268 | 27.3 | 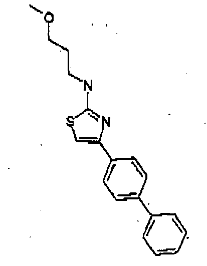 |
| **r48** | MFCD00206686 | 1.6 ± 0.15 | 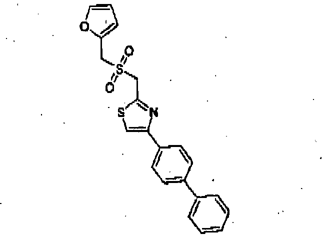 |
| **r49** | MFCD01312821 | 1.5 ± 0.17 | 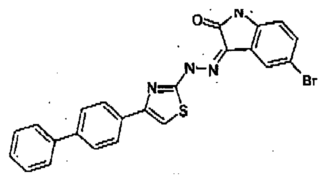 |
| **r66** | MFCD02050262 | 3.6 ± 0.5 | 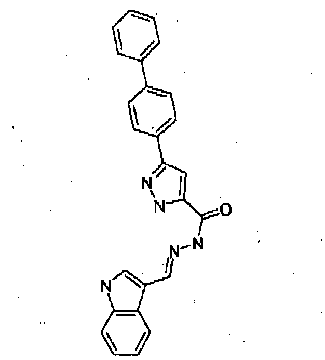 |
| **r67** | MFCD00366058 | 4.7 ± 0.7 | 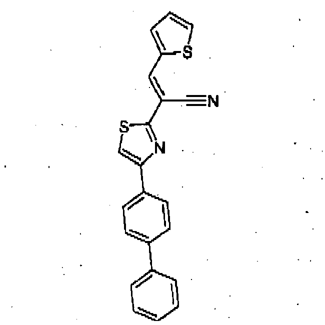 |
| **r67** | MFCD00366058 | 4.7 ± 0.7 | 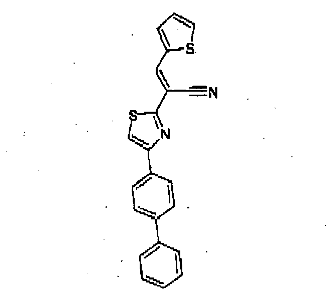 |
| **r83** | MFCD00110238 | 11.7 | 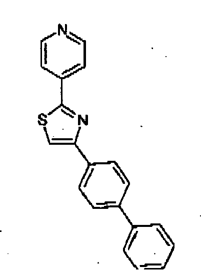 |
| **r85** | MFCD00096941 | 16 ± 4.8 | 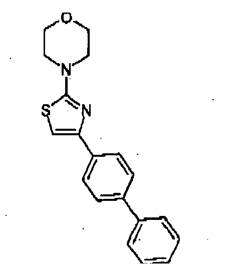 |
| **r84** | MFCD00806357 | 11 ± 1.3 | 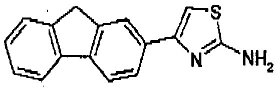 |
| | **Example 1** **Compound No.** | | |
| **r114** | **(1)** | 0.75 ± 0.13 | |
| **r218** | **(2)** | 1.3 ± 0.5 | |
| **r236** | **(4)** | 1.3 ± 0.4 | |
| **r237** | **(6)** | 31 ± 5.7 | |
| **r239** | **(7)** | 20 ± 2.3 | |
| **r113** | **(64)** | 57 ± 5 | |
| **r116** | **(12)** | 27 ± 1.6 | |
| **r117** | **(13)** | 58 ± 5.8 | |
| **r120** | **(14)** | 12 ± 1.2 | |
| **r121** | **(65)** | 42 ± 3.3 | |
| **r122** | **(66)** | 37 ± 2.7 | |
| **r124** | **(67)** | 11 ± 0.72 | |
| **r127** | **(15)** | 4.6. | |
| **r128** | **(16)** | 6.7 | |
| **r200** | **(17)** | 4.8 ± 0.7 | |
| **r201** | **(68)** | 17 ± 1.5 | |
| **r235** | **(22)** | 21 ± 2.5 | |
| **r238** | **(21)** | 9.6 ± 1.1 | |
| **r262** | **(20)** | 3.7 ± 0.5 | |
| **r86** | **(42)** | 6.7 ± 0.5 | |
| **r87** | **(43)** | 4.3 ± 0.3 | |
| **r88** | **(39)** | 1.4 ± 0.1 | |
| **r89** | **(69)** | 1.3 ± 0.2 | |
| **r104** | **(41)** | 2.9 ± 0.2 | |
| **r105** | **(29)** | 8.2 ± 0.3 | |
| **r106** | **(37)** | 9.5 ± 0.4 | |
| **r108** | **(32)** | 7.0 ± 0.3 | |
| **r156** | **(57)** | 82 ± 9 | |
| **r158** | **(59)** | 10 ± 0.73 | |
| **r169** | **(60)** | 17 ± 1.4 | |
| **r170** | **(62)** | 14 ±1.6 | |
| **r233** | **(70)** | 2.1 ± 0.7 | |

### 3. Inhibition of the proliferation of NPM/ALK transformed cells

### Method: Tritiated thymidine uptake cell proliferation assay

BaF3 cells, transformed with the oncogenic fusion protein NPM/ALK, were seeded in U-bottomed 96-well plates at 10 000 cells/well in a volume of 100 µL in supplemented medium. Serial dilutions of inhibitors were added to the appropriate wells and volumes adjusted to 200 µL. Controls were treated with the equivalent volume of vehicle, DMSO, alone. Plates were incubated at 37°C for 72 h. ³[H]-thymidine (1 µCi/well) was added for the last 16 h of incubation. Cells were harvested on to glass filters and ³[H]-thymidine incorporation was measured using a β scintillation counter (1430 MicroBeta, Wallac, Turku, Finland). The 50% inhibitory concentration (IC₅₀) was defined as the concentration of inhibitor that gave a 50% decrease in ³[H]-thymidine uptake compared with controls.

### Results for proliferation assay

**Table 2: IC₅₀ values on the proliferation of BaF3 cells transformed with NPM/ALK**

| compound | | Identifier | | IC₅₀ (µM) | |
|---|---|---|---|---|---|
| **r78** | | MFCD01934431 | | 7.6 | |
| **r79** | | MFCD00113424 | | 33 | |
| **r80** | | MFCD00113296 | | 31 | |
| | | | | | |
| **r49** | | MFCD01312821 | | 12 | |
| **r66** | | MFCD02050262 | | 4.8 | |
| | | | | | |

| | Compound | | IC₅₀ (µM) | | |
|---|---|---|---|---|---|
| | **r89** | | 36 | | |
| | **r104** | | 7 | | |
| | **r105** | | 2.3 | | |
| | **r106** | | 1.9 | | |

### 4. Abl T315I mutant kinase inhibitory activity

### Method: ELISA-based in vitro kinase assay

Recombinant Abl T315I protein was expressed in Sf9 cells using the pBlueBacHis2C baculovirus expression vector. Abl T315I was purified using an anion exchange Fast Flow Q-sepharose column (Amersham-Pharmacia Biotech) followed by HiTrap™-nickel affinity column (Amersham-Pharmacia Biotech). Purified Abl T315I was used in the ELISA-based kinase assay to screen inhibitors as described above. The kinase reaction was performed in the presence of 50 mM Tris pH 7.5, 1 mM MnCl₂, 5 mM MgCl₂, 0.3 mM ATP, peptide substrate (ARDIYRASFFRKGGCAMLPVK) and purified Ab1 T315I. The concentration of the test solution showing 50% inhibition as compared with the control was expressed as IC₅₀.

**Table 3: IC₅₀ values on Abl T315I**

| | Compound | | IC₅₀ (µM) | | |
|---|---|---|---|---|---|
| | **r87** | | 6.43 ± 0.43 | | |
| | **r88** | | 1.45 ± 0.35 | | |
| | **r104** | | 7.0 ± 1.4 | | |
| | **r114** | | 2.1 | | |
| | | | | | |

| compound | | Identifier | | IC₅₀ (µm) | |
|---|---|---|---|---|---|
| **r37** | | MFCD01765092 | | 3.3 ± 0.81 | |

### REFERENCES

1. Rabbitss, T.H. Nature, 1994, 372, 143.
2. Ben-Neriah, Y., Daley, G.Q., Mes-Masson, A.M., Witte, O.N. & Baltimore, D. Science. 1986, 233, 212.
3. Gambacorti-Passerini, C.B., Gunby, R.H., Piazza, R., Galietta, A., Rostagno, R. & Scapozza, L. Lancet Oncol. 2003, 4, 75-85.
4. Morris, S. W; Kirstein, M.N.; Valentine, M.B.; Dittmer, K.G.; Shapiro, D.N.; Saltman, D.L.; Look; A.T. Science, 1994, 263,1281-1284.
5. Shah, N.P., Tran, C., Lee, F.Y., Chen, P., Norris, D. & Sawyers, C.L. Science 2004 305, 399-401.
6. Puttini, M.; Coluccia, A.M.; Boschelli, F.; Cleris, L.; Marchesi, E.; Donella-Deana, A.; Ahmed, S.; Redaelli, S.; Piazza, R.; Magistroni, V.; Andreoni, F.; Scapozza, L.; Formelli, F.; Gambacorti-Passerini, C. Cancer Res. 2006, 66, 11314-11322.
7. Weisberg, E., Manley, P.W., Breiteristein, W., Bruggen, J., Cowan-Jacob, S.W., Ray, A., Huntly, B., Fabbro, D., Fendrich, G., Hall-Meyers, E., Kung, A.L., et al. Cancer Cell. 2005 7, 129-141.

## Claims

1. A compound of formula (I): 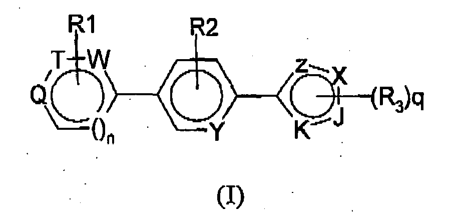 wherein Q, T, W, K, J, Y, X, Z are independently selected from C, N, S, O, provided that the corresponding rings are (hetero)aromatic;
n = 0 or 1;
q=1 or 2;
R1 is selected from: 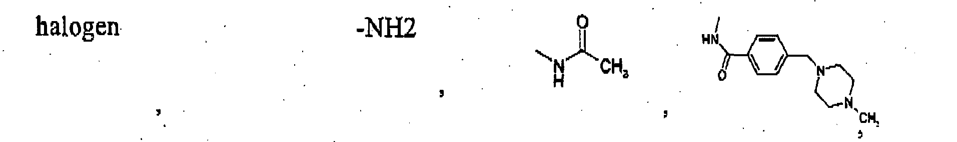 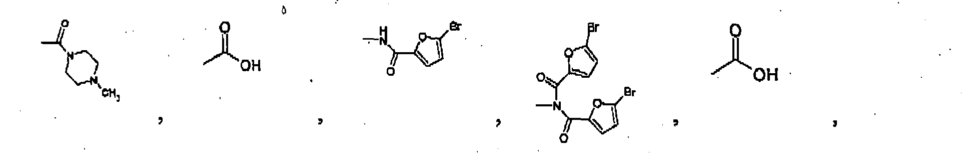 R2 is hydrogen or halogen,
R3 is selected from: 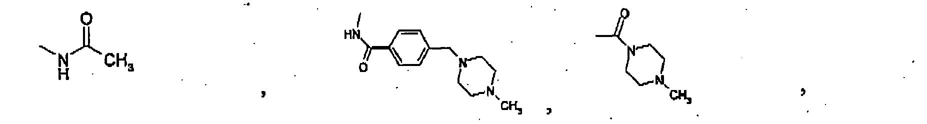 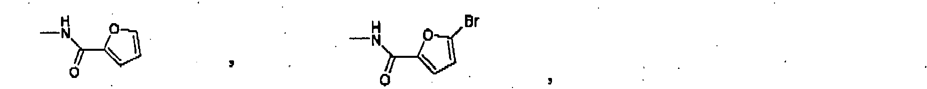 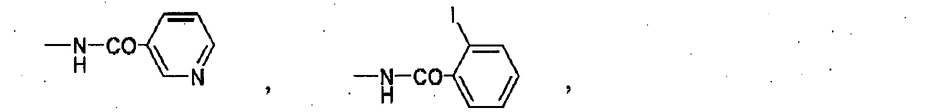 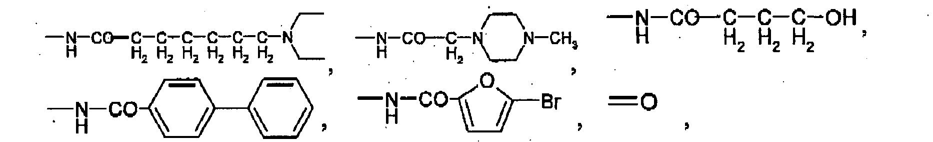 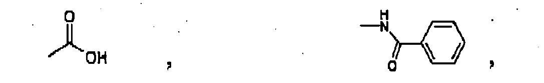 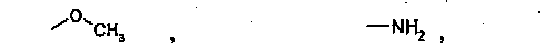 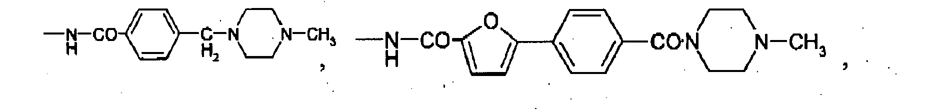 or the moiety 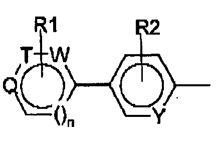 in formula (I) forms a group 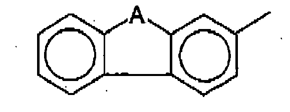 wherein A is -CH2- or -NH-.

2. A compound according to claim 1, of formula (Ia): 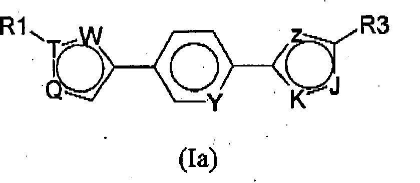 wherein
R1 is selected from 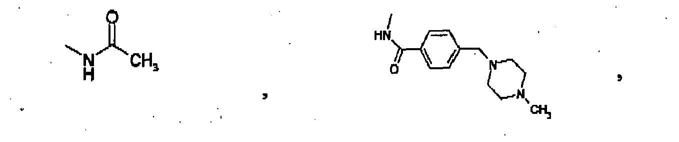 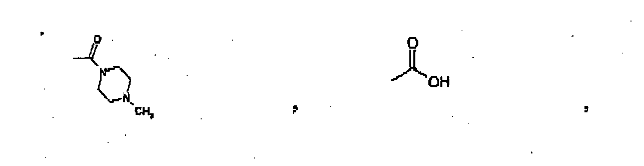 R3 is selected from: 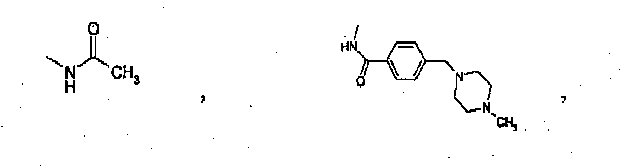 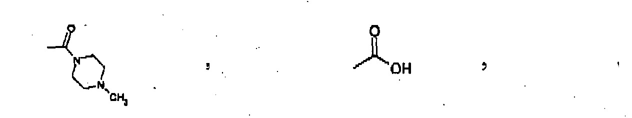 W, T, Q, Y, K, J and Z are as defined above.

3. A compound according to claim 1, of formula (IIa)
wherein: 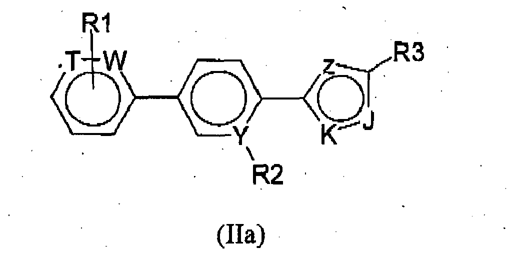 R1 is selected from 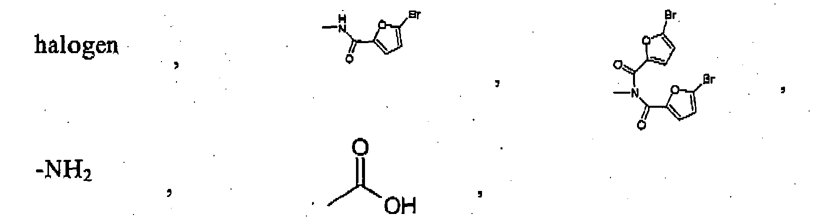 R2 is halogen,
R3 is selected from: 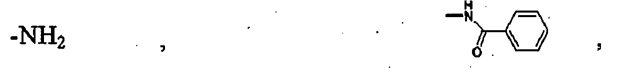 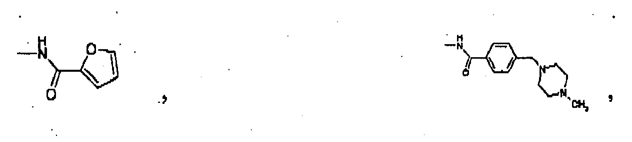 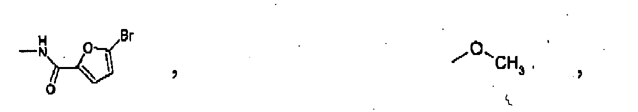 T, W, Y, K, J, Z are as defined above.

4. A compound according to claim 1, of formula (IIIa) 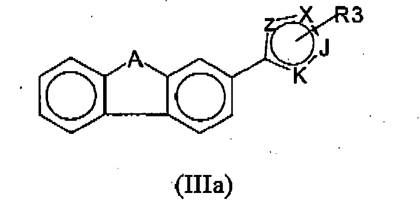 wherein A is -CH2- or -NH-,
R3 is selected from: 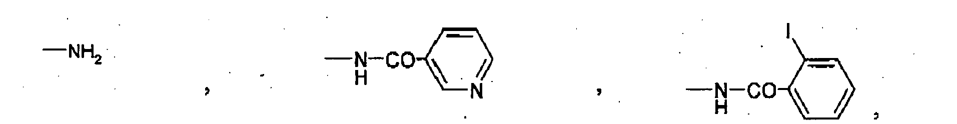 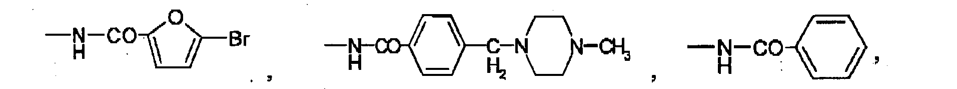 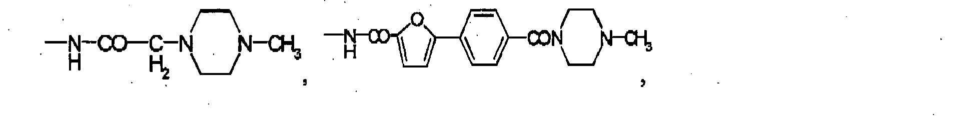 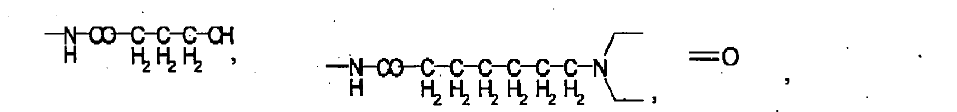 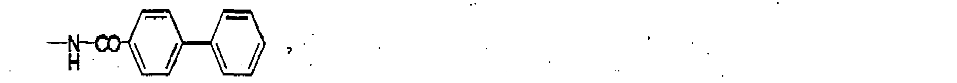 X, Z, J and K are as defined above.

5. A compound according to claim 1, which is selected from the group consisting of:
- N,N'-(4,4'-(1,4-phenylene)bis(thiazole-4,2-diyl))bis(4-((4-methylpiperazin-1-yl)methyl)benzamide)
- 1,4-di(furan-3-yl)benzene (r236)
- (5-{4-[5-(4-Methyl-piperazine-1-carbonyl)-furan-2-yl]-phenyl}-furan-2-yl)-(4-methyl-piperazin-1-yl)-methanone
- 5,5'-(1,4-phenylene)difuran-2-carboxylic acid
- 5-(2-bromophenyl)-2-(thiazol-2-yl)pyridine
- N-(5-(9H-fluoren-7-yl)thiazol-2-yl)nicotinamide
- N-(5-(9H-fluoren-7-yl)thiazol-2-yl)-2-iodobenzamide
- N-(5-(9H-fluoren-2-yl)thiazol-2-yl)-5-bromofuran-2-carboxamide
- 5-[4-(4-Methyl-piperazine-1-carbonyl)-phenyl]-furan-2-carboxylic acid [5-(9H-fluoren-2-yl)-thiazol-2-yl]-amide
- N-(5-(9H-fluoren-7-yl)thiazol-2-yl)-2-(4-methylpiperazin-1-yl)acetamide
- N-(5-(9H-fluoren-7-yl)thiazol-2-yl)-4-hydroxybutanamide
- 4-(9*H*-Carbazol-2-yl)-thiazol-2-ylamine
- *N*-[4-(9*H*-Carbazol-2-yl)-thiazol-2-yl]-benzamide
- iphenyl-4-carboxylic acid 4-(9*H*-carbazol-2-yl)-thiazol-2-yl]-amide
- 5-Bromo-furan-2-carboxylic acid [4-(9*H*-carbazol-2-yl)-thiazol-2-yl]-amide

6. A compound selected from the group consisting of: 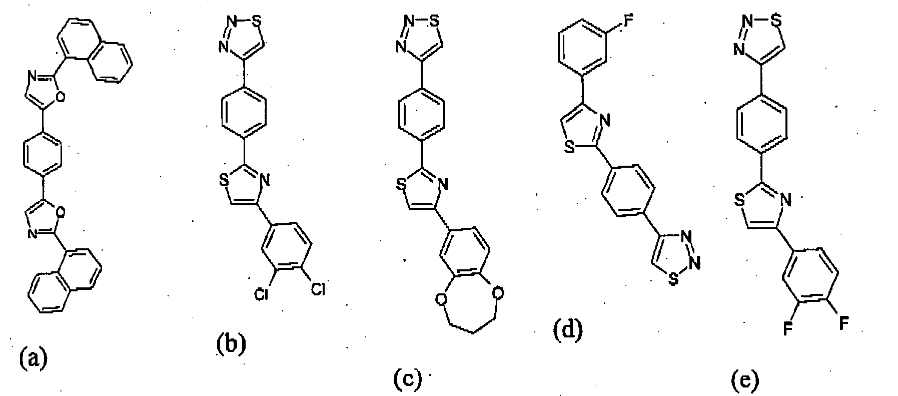 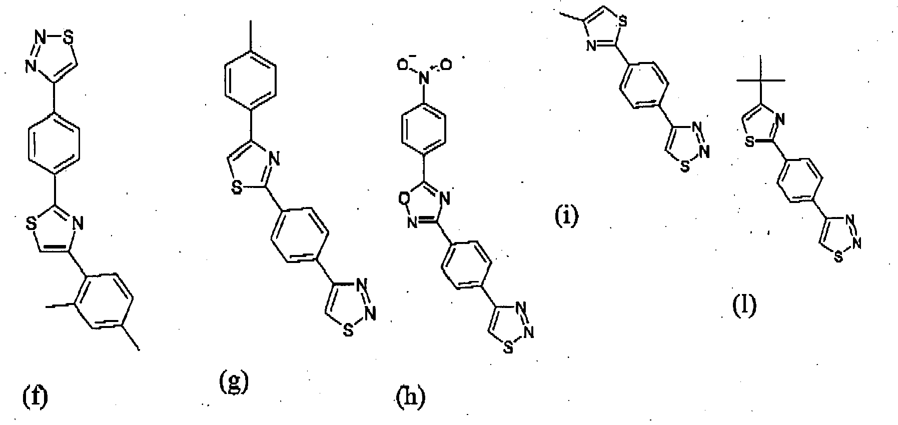 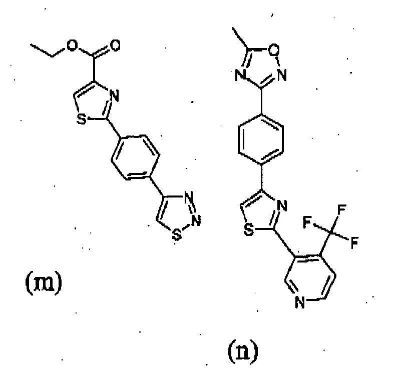 for use as a therapeutic agent.

7. A pharmaceutical composition containing a compound according to any preceding claim in association with a physiologically acceptable vehicle or excipient.

8. A compound according to any one of claims 1-6 or a composition according to claim 7, for use in the treatment of tumors.

9. A compound or composition as claimed in claim 8, for use in the treatment of Anaplastic Lymphoma Kinase - associated or Bcr-Abl- associated tumors.

10. A compound or composition as claimed in claim 8, for use in the treatment of anaplastic large cell lymphoma, diffuse large B cell lymphoma, inflammatory myofibroblastic tumors, chronic myeloid leukemia or Ph+ acute lymphoblastic leukemia.
